# EUROPÄISCHE PATENTSCHRIFT

(11) **EP 3 708 208 B1**
(45) Veröffentlichungstag und Bekanntmachung des Hinweises auf die Patenterteilung: **08.06.2022**
(21) Anmeldenummer: 20000058.6
(22) Anmeldetag: 10.02.2020
(51) Int. Cl.: A61M 16/00, A61M 16/10

(54) **VORRICHTUNG ZUR BEATMUNG MIT PNEUMATIKSTRANG**
VENTILATION DEVICE WITH PNEUMATIC TRAIN
DISPOSITIF DE RESPIRATION À L'AIDE D'UN CANAL PNEUMATIQUE

(30) Priorität: 20.02.2019 DE 102019104332
(43) Veröffentlichungstag der Anmeldung: 16.09.2020
(73) Patentinhaber: Löwenstein Medical Technology S.A., 2557 Luxembourg (LU)
(72) Erfinder: Sepke, Heiko, 22952 Lütjensee (DE); Salem-Cherif, Mohamed, 22529 Hamburg (DE); Gardein, Joachim, 38430 Icod de los Vinos Tenerife / Islas Canarias (ES); Hahn, Henry, 22525 Hamburg (DE)
(74) Vertreter: Marx, Thomas

(56) Entgegenhaltungen:
- WO-A1-2018/074935
- WO-A2-2006/107818
- WO-A2-2010/044037
- WO-A2-2014/184377
- US-A1- 2008 257 346
- US-A1- 2013 206 140
- US-A1- 2017 082 116
- US-A1- 2017 340 847

## Beschreibung

Die vorliegende Erfindung betrifft eine Vorrichtung zur Beatmung umfassend ein Gehäuse umfassend eine Pneumatikeinheit und zumindest eine weitere Komponente, insbesondere einen Atemgasantrieb und zumindest eine Messeinheit, sowie eine Exspirationseinheit, einen Akkumulator, eine Steuereinheit und einen Kühllüfter.

Derartige Vorrichtungen zur Beatmung werden typischerweise zur Energieversorgung an ein Versorgungsnetz angeschlossen und über einen Beatmungsschlauch mit einer Beatmungsmaske oder einem andren Patienteninterface verbunden. Ein Patient legt die Beatmungsmaske während der Durchführung der Beatmung im Gesichtsbereich an.

Beispielsweise ist bekannt, derartige Vorrichtungen zur Beatmung zur Durchführung einer CPAP-Therapie, einer APAP-Therapie oder einer Bilevel Beatmung zu verwenden. Solche Vorrichtungen zur Beatmung werden auch in der Notfalltherapie, Hustentherapie und in der Klinik eingesetzt. Grundsätzlich können von der Steuerung des Gerätes beliebige Beatmungsabläufe vorgegeben werden.

Es ist bekannt, Vorrichtung zur Beatmung/Beatmungsgeräte mit einem Leckageschlauchsystem, einem Einschlauchsystem oder einem Doppelschlauchsystem zu betreiben. Um von einem Leckageschlauchsystem oder einem Einschlauchsystem auf ein Doppelschlauchsystem zu wechseln, offenbaren die bekannten Beatmungsgeräte einen Umbau eines Anschlussbereiches des Schlauchsystems am Beatmungsgerät.

Bei Verwendung eines Leckagesystems wird über ein Ausatemsystem die CO2-haltige Ausatemluft kontinuierlich ausgespült. Während bei einem Ventilsystem die Ausatmung über das Patientenventil gesteuert wird. Bei Verwendung des Einschlauchsystems mit Patientenventil entweicht die Ausatemluft des Patienten über das Patientenventil in die Umgebung. Das Gerät steuert das Patientenventil. Bei Verwendung des Doppelschlauchsystems mit Patientenventil führt zusätzlich ein Ausatemschlauch die Ausatemluft durch das Beatmungsgerät in die Umgebungsluft ab.

Aus dem Stand der Technik ist beispielsweise durch die US 2017/0340847 A1 ein Gerät zur Beatmung bekannt, welches ein aus dem Gehäuse entnehmbares Gasleit-Modul zur Führung des Atemgases innerhalb des Gerätes beinhaltet. Auch werden weitere austauschbare Komponenten vorgestellt.

Die US 2013/0206140 A1 beschreibt ebenfalls ein Beatmungsgerät, welches eine modulare Bauweise aufweist, bei dem unter anderem auch ein Tank als Teil der Pneumatik austauschbar dargestellt wird.

In der US 2017/0082116 A1 wird ein Beatmungsgerät beschrieben, bei dem einzelnen Komponenten innerhalb des Atemgasweges, beispielsweise Filterelemente, getauscht werden können. Auch wird dazu eine modulare Gasleitungseinheit beschrieben.

Neben einem zum Teil modularen Aufbau mit entnehmbaren Teilen der Pneumatik, beschreibt die WO 2014/184377 A2 auch die Anordnung eines Kühllüfters innerhalb des Gerätegehäuses, welcher eine Strömung von erwärmter Luft innerhalb des Gehäuses erzeugt.

Aufgabe der vorliegenden Erfindung ist es, eine Vorrichtung der einleitenden Art derart zu konstruieren, welche ein erhöhtes Maß an Sicherheit und Zuverlässigkeit bietet.

Diese Aufgabe wird durch eine Vorrichtung zur Beatmung gemäß dem Anspruch 1 gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Die vorliegende Erfindung betrifft eine Vorrichtung zur Beatmung umfassend ein Gehäuse, eine Benutzerschnittstelle und eine Pneumatikeinheit, wobei die Pneumatikeinheit zumindest je eine der Komponenten Atemgasantrieb, Messeinheit, Steuereinheit und Schalleinheit umfasst, wobei die Pneumatikeinheit als Pneumatikstrang ausgebildet ist, wobei der Pneumatikstrang einen Atemgasweg von einem Geräteeingang zu einem Geräteausgang des Gehäuses ausbildet und wobei zumindest eine Komponente der Pneumatikeinheit oder der Pneumatikstrang aus dem Gehäuse entnehmbar ist.

Die vorliegende Erfindung betrifft auch eine Vorrichtung zur Beatmung umfassend ein Gehäuse umfassend eine Pneumatikeinheit und zumindest eine weitere Komponente, insbesondere einen Atemgasantrieb und zumindest eine Messeinheit, sowie eine Exspirationseinheit, einen Akkumulator, eine Steuereinheit und einen Kühllüfter.

Die Messeinheit kann ein Fluss-, Druck-, Volumen-, Gas-, oder Sauerstoffsensor sein oder einen solchen umfassen.

Erfindungsgemäß ist die Pneumatikeinheit als Pneumatikstrang ausgebildet, wobei der Pneumatikstrang von einem Geräteeingang zu einem Geräteausgang des Gehäuses ausgebildet und angeordnet ist und einen Trägerrahmen umfasst, auf dem zumindest eine Komponente, insbesondere der Atemgasantrieb, zumindest eine Schalleinheit, zumindest eine Flussmessstrecke, zumindest eine Messeinheit und der Kühllüfter angeordnet sind, wobei der Pneumatikstrang aus dem Gehäuse entnehmbar ist. In der Regel umfasst der Pneumatikstrang zumindest den Atemgasantrieb, den Kühllüfter und/oder eine Sauerstoffeinheit/O2-Kartusche.

Der Trägerrahmen ist eingerichtet, den Pneumatikstrang umfassend zumindest eine Komponente, insbesondere den Atemgasantrieb, zumindest eine Schalleinheit, zumindest eine Flussmessstrecke, zumindest eine Messeinheit und zumindest den Kühllüfter, aufzunehmen. Der Trägerrahmen ist eingerichtet, in der Vorrichtung, in dem Gehäuse anordenbar zu sein. In der Regel ist der Trägerrahmen an dem Gehäuse zumindest bereichsweise angeordnet. Beispielsweise kann der Trägerrahmen mit dem Gehäuse verschraubt sein oder der Trägerrahmen kann in eine Aufnahme des Gehäuses einbringbar ausgebildet und eingerichtet sein.

Der Atemgasantrieb kann ein Gebläse, ein Ventil, eine Sauerstoff-Quelle (Hochdruck) oder eine Luftdruckquelle (Hochdruck) oder eine Kombination aus den Vorgenannten sein. Der Atemgasantrieb ist beispielsweise über zumindest zwei, insbesondere drei Aufhängepunkte frei schwingend in dem Pneumatikstrang angeordnet.

Die Steuereinheit der Vorrichtung umfasst in der Regel zumindest eine Speichereinheit und eine Auswerteeinheit. Die Speichereinheit ist eingerichtet, Messwerte, Informationen und/oder Parameter zu speichern und für Auswertungen durch die Auswerteeinheit bereitzustellen. Die Auswerteeinheit ist eingerichtet, die Messwerte, Informationen und/oder Parameter miteinander oder mit externen Daten zu vergleichen. Die Steuereinheit ist eingerichtet, Daten von Komponenten der Vorrichtung, insbesondere einer Messeinheit der Flussmessstrecke zu empfangen, zu speichern und zu analysieren. Optional ist die Steuereinheit eingerichtet, Daten Messwerte, Informationen und/oder Parameter an eine digitale Schnittstelle der Vorrichtung zu übermitteln.

Die Vorrichtung ist insbesondere auch für die Verwendung in der pädiatrischen Beatmung eingerichtet. Die Vorrichtung umfasst hinterlegte Beatmungsmodi. Insbesondere umfasst die Vorrichtung zumindest einen High-Flow-Modus und zumindest einen PEEP-Steuerungsmodus. In der Regel ist die Steuereinheit der Vorrichtung eingerichtet, die Beatmungsmodi, Frequenzen, Trigger und Flüsse der Vorrichtung einzustellen.

Der Pneumatikstrang ist in der Regel einteilig aus dem Gehäuse entnehmbar. Dabei ist der Pneumatikstrang zusammen mit dem Trägerrahmen aus dem Gehäuse entnehmbar. Einteilig bedeutet, dass der Trägerrahmen umfassend den Pneumatikstrang in einem Stück aus dem Gehäuse entnehmbar und einsetzbar ist. Dies bietet den Vorteil, dass bei Wartungs- und Reparaturaufgaben der Pneumatikstrang schnell und gesichert aus dem Gehäuse entnehmbar ist.

In der Regel werden insbesondere aufbereitbare/wiederverwendbare Komponenten auf/in dem Pneumatikstrang angeordnet. Dies bietet den Vorteil, dass aufbereitbare/wiederverwendbare Komponenten schnell und einteilig aus der Vorrichtung entnehmbar sind. Typischerweise kann der Pneumatikstrang einteilig aus dem Gehäuse entnommen werden und einteilig in die Aufbereitung gegeben werden. Alternativ kann der Pneumatikstrang einteilig aus dem Gehäuse entnommen werden, wobei in ausgebautem Zustand die Komponenten einzeln aus dem Pneumatikstrang entnehmbar sind. Dies bietet den Vorteil, dass die Komponenten auch einzeln in die Aufbereitung gegeben oder ausgetauscht werden können. In der Regel ist zumindest der Atemgasantrieb eingerichtet, mit einer Tauchdesinfektion desinfizierbar zu sein. Optional sind die Komponenten Einweg-Komponenten oder die Komponenten sind eingerichtet autoklavierbar, desinfizierbar oder sterilisierbar zu sein.

Die voranstehend beschriebene Vorrichtung weist insgesamt den Vorteil auf, dass eine Beatmung eines Patienten ermöglicht wird, wobei eine Mobilität des Patienten aufrechterhalten werden kann. Beispielsweise kann die Vorrichtung an einem Rollstuhl angebracht sein. Die Vorrichtung umfasst zudem eine Absaugungsfunktion und einen Hustenmodus. Die Vorrichtung kann an verschiedene Schlauchsysteme angepasst werden, ohne dass ein Umbau des Anschlussbereiches des Schlauchsystems am Beatmungsgerät erfolgt. Des Weiteren weist die voranstehende Vorrichtung den Vorteil auf, dass diese schnell und einfach zu reinigen ist, einen schmalen Aufbau mit optionaler Ausrichtung aufweist und durch die Schalleinheiten in Betrieb äußerst leise ist.

In einer Weiterbildung der Erfindung sind aus dem Pneumatikstrang in aus dem Gehäuse ausgebauten Zustand Komponenten entnehmbar. Dies bietet den Vorteil, dass ebenfalls einzelne verbrauchte Komponenten oder beschädigte Komponenten schnell und einfach aus der Vorrichtung und dem Pneumatikstrang entnehmbar sind. Zudem wird das Risiko für Beschädigungen anderer Komponenten während der Wartungsarbeiten reduziert. In der Regel weist der Pneumatikstrang zumindest ein Rückschlagventil mit zumindest einem Bypass auf.

In Ausgestaltung ist der Pneumatikstrang in dem Gehäuse der Vorrichtung von zumindest einer viskoelastischen oder elastomeren Aufhängung gehaltert. In der Regel wird der Pneumatikstrang umfassend den Trägerrahmen an zumindest zwei Punkten/Bereichen in dem Gehäuse von zumindest einer viskoelastisch oder elastomeren Aufhängung gehaltert. Die viskoelastische Aufhängung vereint die Funktionen Lagerung, Schalldämpfung und Anbindung des Pneumaitkstrangs bzw. des Atemgasantriebs in dem Gehäuse.

In einer Weiterbildung ist der Pneumatikstrang im Kontaktbereich des Pneumatikstrangs zum Gehäuse, insbesondere im Bereich eines Atemluftausgangs, eines Kühllufteingangs und des Kühllüfters, gehaltert. Dies bietet den Vorteil, dass insbesondere die Bereiche des Pneumatikstrangs viskoelastisch gehaltert werden, die durch eine Berührung mit dem Gehäuse oder durch ihre Beschaffenheit eine erhöhte Lautstärkenentwicklung oder Vibrationsentwicklung aufweisen.

In einer weiteren Weiterbildung weist das Gehäuse eine Dachwand, zumindest eine Bodenwand und zumindest zwei Seitenwände auf, wobei der Geräteeingang und der Geräteausgang an zwei sich nicht gegenüberliegenden Seitenwänden des Gehäuses ausgebildet sind. In der Regel weist das Gehäuse eine rechteckige Form auf. Alternativ kann das Gehäuse eine runde, kreisförmige oder halbkreisförmige Ausgestaltung sowie jede andere geometrische Form aufweisen. Optional sind der Geräteeingang und der Geräteausgang an zwei sich nicht gegenüberliegenden Seiten des Gehäuses ausgebildet. Optional kann der Geräteeingang im rechten Winkel zu dem Geräteausgang angeordnet sein.

Die Ausgestaltung des Geräteeingangs und des Geräteausgangs an zwei sich nicht gegenüberliegenden Seitenwänden des Gehäuses bietet den Vorteil, dass die Vorrichtung durchströmende Luft, die zum Patienten geführt werden soll, einen längeren Weg durch das Gehäuse der Vorrichtung geführt werden kann, wobei eine mehrfache Umlenkung der Luft erzielt werden kann, wodurch Schall reduziert wird. Dies trägt zu einer besonders leisen Betriebsweise der Vorrichtung bei.

Die Dachwand ist in der Regel eingerichtet, eine Benutzerschnittstelle, insbesondere ein Bedien- und Anzeigeelement aufzunehmen. Die Anordnung der Benutzerschnittstelle im Bereich der Dachwand bietet den Vorteil, dass der Benutzer frei auf die Bedienoberfläche der Benutzerschnittstelle zugreifen kann.

In einer weiteren Weiterbildung ist zumindest eine Seitenwand als optionale Bodenwand eingerichtet. Durch die Ausgestaltung zumindest einer Seitenwand als optionale Bodenwand kann die Vorrichtung neben der horizontalen Ausrichtung auf der Bodenwand auch vertikal auf der optionalen Bodenwand aufgestellt werden. Durch die Einrichtung zumindest einer Seitenwand als optionale Bodenwand wird die Standfläche der Vorrichtung verkleinert und somit der von der Vorrichtung benötigte Platz verringert. Zudem wird die Orientierung der Benutzerschnittstelle derart geändert, dass ein Benutzer die Vorrichtung auch im Liegen problemlos einsehen und bedienen kann. In der Regel ist die optionale Seitenwand eine Seitenwand, die einer Seitenwand, die eingerichtet ist, Bedienelemente aufzunehmen, gegenüberliegt.

Die Vorrichtung ist auch dadurch gekennzeichnet, dass sowohl in der Bodenwand, als auch in der als optionale Bodenwand eingerichteten Seitenwand, kein Kühllufteingang oder Kühlluftausgang oder Geräteeingang oder Geräteausgang angeordnet ist.

In Ausgestaltung ist die Bodenwand des Gehäuses abnehmbar und der Pneumatikstrang über eine Öffnung der Bodenwand einteilig entnehmbar. Dies bietet den Vorteil, dass nur eine Wand der Vorrichtung des Gehäuses der Vorrichtung entfernt werden muss, um den Pneumatikstrang aus dem Gehäuse zu entfernen. Dies trägt zu einer schnellen und vereinfachten Wartung des Gerätes bei.

In einer Weiterbildung der Erfindung sind der Akkumulator und der Atemgasantrieb derart im Gehäuse angeordnet, dass bei einer Ausrichtung der Vorrichtung sowohl auf der Bodenwand als auch auf der als optionale Bodenwand eingerichteten Seitenwand durch den Akkumulator und den Atemgasantrieb ein Schwerpunkt ausgebildet ist. Dies bietet den Vorteil, dass durch die Anordnung schwerer Komponenten der Vorrichtung jeweils in einem unteren Bereich des Gehäuses sowohl bei einer Orientierung auf der Bodenwand als auch auf der optionalen Bodenwand die Standfestigkeit der Vorrichtung gewährleistet werden kann.

In einer weiteren Weiterbildung weist der Pneumatikstrang zumindest zwei Schalleinheiten auf, wobei zumindest eine erste Schalleinheit auf einer Saugseite des Pneumatikstrangs und eine zweite Schalleinheit auf einer Druckseite des Pneumatikstrangs angeordnet ist. Durch die Einrichtung einer Schalleinheit auf der Saugseite und einer Schalleinheit auf der Druckseite des Pneumatikstrangs kann besonders effektiv eine Schallentwicklung im Pneumatikstrang reduziert werden.

Optional umfasst die Saugseite der Vorrichtung zumindest zwei Schalleinheiten. Beispielsweise kann eine erste Schalleinheit auf der Saugseite in Strömungsrichtung dem Kühllufteingang nachgeschaltet angeordnet sein und eine zweite Schalleinheit auf der Saugseite direkt dem Atemgasantrieb vorgeschaltet angeordnet sein. Die erste Schalleinheit der Saugseite und die zweite Schalleinheit der Saugseite können hintereinander oder getrennt voneinander angeordnet sein.

In Ausgestaltung ist die Schalleinheit auf der Saugseite an einer Aufnahme des Atemgasantriebs angeordnet und umfasst eine Gebläsekappe, einen Schaumhalter, zumindest einen Absorberschaum, eine Entkopplungsvorrichtung und zumindest einen Ringschaum. Die Entkopplungsvorrichtung ist eingerichtet, den Atemgasantrieb von der saugseitigen Schalleinheit zu entkoppeln. Dies trägt zu einer verringerten Geräuschentwicklung bei. Die saugseitige Schalleinheit umfasst in der Regel einen Sauerstoffeinlass der O2-Kartusche.

Der Ringschaum ist eingerichtet, die Atemluft die durch den Ringschaum strömt, gerade zu führen. Dies bietet den Vorteil, das Turbulenzen hinter der Gebläsekappe minimiert werden und gleichzeitig der Luftschall der Strömung und des Atemgasantriebs absorbiert wird. Die Schalleinheit mit ihren Absorberschäumen bildet eine komplexe Luftführung mit mehrfacher Luftumlenkung bei gleichzeitiger Schalldämmung aus.

In einer weiteren Ausgestaltung ist der Pneumatikstrang eingerichtet und angeordnet, eine Atemluft innerhalb des Pneumatikstrangs derart zu führen, dass verschiedene Luftführungen für verschiedene Schlauchsysteme wählbar sind. Insbesondere ist die Vorrichtung eingerichtet, mit einem Leckageschlauchsystem, einem Einschlauchsystem oder einem Doppelschlauchsystem betrieben zu werden. Die derart eingerichtete Vorrichtung bietet den Vorteil, dass zwischen den einzelnen Schlauchsystemen gewechselt werden kann ohne einen Umbau an der Vorrichtung selbst vorzunehmen.

In Ausgestaltung bildet die Vorrichtung eine Kühleinheit aus, wobei die Kühleinheit einen Kühllufteingang und einen Kühlluftausgang aufweist, wobei zwischen dem Kühllufteingang und dem Kühlluftausgang ein Kühlluftweg eingerichtet und ausgebildet ist. Die Kühleinheit besteht somit aus einzelnen Komponenten, die einen Kühlluftstrom über einen Kühlluftweg führen. Der Kühlluftweg ist in der Regel derart eingerichtet, dass er frei in dem Gehäuse der Vorrichtung geführt ist, wobei eine Hauptströmungsrichtung des Kühlluftwegs in der Regel von einer Seitenwand zu einer gegenüberliegenden Seitenwand geführt ist. Alternativ kann der Kühlluftweg von einer Seitenwand zu einer nicht gegenüberliegenden Seitenwand geführt sein.

In einer Weiterbildung ist der Kühllüfter derart in dem Gehäuse angeordnet, das ein Luftstrom von dem Kühllufteingang zumindest über die Steuereinheit und den Atemgasantrieb in Richtung des Kühlluftausgangs geführt ist. Der Kühllüfter ist in der Regel eingerichtet, Kühlluft von dem Kühllufteingang, der in der Regel in einer gegenüberliegenden Seitenwand der Seitenwand, in dem der Kühllüfter angeordnet ist, angeordnet ist, anzusaugen. Dabei ist der Kühllüfter eingerichtet, die Kühlluft durch das Innere des Gehäuses der Vorrichtung über die einzelnen Komponenten des Pneumatikstrangs zu führen.

Der Kühllüfter ist in der Regel regelbar eingerichtet. Regelbar bedeutet, dass die Umdrehung bzw. Laufleistung des Kühllüfters stufenlos einstellbar ist. Der Kühllüfter kann eingerichtet sein, automatisch regelbar zu sein. Typischerweise umfasst der Kühllüfter zumindest einen Temperatursensor, wobei der Kühllüfter eingerichtet ist, basierend auf einer durch den Temperatursensor erfassten Temperatur in dem Kühllüfter oder basierend auf einer Kennlinie entsprechend eines Beatmungsmodus steuerbar/regelbar zu sein. Der Kühllüfter ist eingerichtet, Kühlluft/Umgebungsluft von einem Kühllufteingang anzusaugen, wobei die Kühlluft über Luftleitbleche im Inneren des Gehäuses der Vorrichtung gezielt führbar ist.

Erfindungsgemäß sind zumindest die Steuereinheit, der Atemgasantrieb und eine Hauptplatine in dem Kühlluftweg angeordnet, wobei die Steuereinheit, der Atemgasantrieb und die Hauptplatine derart in dem Kühlluftweg angeordnet sind, dass die Komponente, die die höchste Wärmeentwicklung im Betrieb aufweist, in Kühlluftwegstromrichtung am Ende des Kühlluftweges angeordnet ist. Dies bietet den Vorteil, dass die Wärme der Komponenten mit der höchsten Wärmeentwicklung eine geringe Strecke des Kühlluftweges geführt wird, bevor sie aus dem Gehäuse ausgeleitet wird. Dadurch wird die Wärme der Steuereinheit, des Atemgasantriebs und der Hauptplatine gezielt kurzfristig in dem Kühlluftweg geführt und schnellstmöglich ausgeleitet. Dies trägt zu einer besonders effektiven Kühlung der Vorrichtung bei.

In einer weiteren Ausgestaltung ist der Akkumulator in dem Kühlluftweg angeordnet. Dies bietet den Vorteil, dass durch die Kühlung des Akkumulators in dem Kühlluftweg ein Ladevorgang des Akkumulators verbessert werden kann.

Erfindungsgemäß ist der Atemgasantrieb in Kühlluftwegstromrichtung am Ende des Kühlluftweges angeordnet. Dies bietet den Vorteil, dass die Komponente mit der in der Regel höchsten Wärmeentwicklung am Ende des Kühlluftweges angeordnet ist. In einer alternativen Ausgestaltung, außerhalb der Erfindung, ist der Akkumulator am Ende des Kühlluftweges angeordnet.

Typischerweise ist der Atemgasantrieb in dem Gehäuse der Vorrichtung freischwingend angeordnet. Dadurch ist der Atemgasantrieb schallentkoppelt in dem Gehäuse gelagert, wodurch eine besonders leise Betriebsweise der Vorrichtung unterstützt wird.

In Ausgestaltung ist der Kühlluftweg gegenläufig und unabhängig zu dem Atemgasweg des Pneumatikstrangs ausgebildet und eingerichtet. Dadurch ist zum einen eine platzsparende Anordnung der Komponenten innerhalb des Gehäuses erzielbar. Zum anderen können dadurch die wärmsten Komponenten des Pneumatikstrangs nah an den Ausgang des Kühlluftwegs angeordnet werden, wodurch eine effektive Kühlung des Gehäuses erzielt wird.

In einer Weiterbildung umfasst die Pneumatikeinheit eine Flussmesstrecke, die zumindest eine Messeinheit umfasst und eingerichtet ist, zumindest einen Parameter eines Atemgases in der Flussmessstrecke zu erfassen, wobei die Flussmessstrecke linear ausgestaltet und eingerichtet ist, wobei an einem Anfang und/oder an einem Ende der Flussmessstrecke ein Gitternetz angeordnet ist und wobei die Flussmesstrecke entnehmbar und aufbereitbar ausgebildet ist. Die Messeinheit ist in der Regel eingerichtet, Daten, Werte, Informationen und Parameter an die Steuereinheit zu übermitteln.

Die Flussmesstrecke ist in der Regel einteilig aus dem Pneumatikstrang entnehmbar. Durch die lineare Ausgestaltung der Flussmessstrecke wird einer turbulenten Strömung der Atemluft innerhalb der Flussmessstrecke vorgebeugt. Die beruhigte Luftführung innerhalb der Flussmesstrecke unterstützt die Messung der Messeinheit in der Flussmessstrecke. Die Flussmesstrecke umfasst zumindest einen Sensor, der eingerichtet ist, einen Fluss der Atemluft zu erfassen. Alternativ kann die Flussmesstrecke weitere Sensoren umfassen, um weitere Messwerte oder Parameter aus der Atemluft zu erfassen. Beispielsweise kann die Flussmessstrecke einen Drucksensor umfassen.

In einer weiteren Weiterbildung ist die mindestens eine Messeinheit aus dem Gehäuse entnehmbar ausgebildet und eingerichtet, wobei die mindestens eine Messeinheit zumindest einen Sensor zur Erfassung zumindest eines Parameters der über den Atemgasweg geführten Atemluft umfasst. Diese Ausgestaltung bietet den Vorteil, dass die Messeinheit austauschbar ist. Somit kann bei einer defekten oder verbrauchten Messeinheit, die Messeinheit einzeln aus dem Pneumatikstrang entnommen werden und aufbereitet oder ausgetauscht werden.

Die Vorrichtung ist auch dadurch gekennzeichnet, dass die Exspirationseinheit aus einer Aufnahme des Gehäuses entnehmbar eingerichtet und ausgebildet ist, wobei die Exspirationseinheit zumindest ein PEEP-Ventil umfasst, welches eine Membran aufweist, die mit einem Steuerdruck beaufschlagt werden kann, um einen Atemgasfluss über die Exspirationseinheit zu sperren oder freizugeben und/oder wobei die die Exspirationseinheit zumindest einen Abgriff für eine Flussmessung aufweist.

Die Vorrichtung zur Beatmung umfassend in einem Aspekt der Erfindung ein Gehäuse, eine Benutzerschnittstelle und eine Pneumatikeinheit, wobei die Pneumatikeinheit zumindest je eine der Komponenten Atemgasantrieb, Messeinheit, Steuereinheit und Schalleinheit umfasst dadurch gekennzeichnet, dass die Pneumatikeinheit als Pneumatikstrang ausgebildet ist, wobei der Pneumatikstrang einen Atemgasweg B von einem Geräteeingang zu einem Geräteausgang des Gehäuses ausbildet, wobei das Gehäuse eine Dachwand, zumindest eine Bodenwand und zumindest zwei Seitenwände aufweist und wobei zumindest eine Seitenwand als optionale Bodenwand eingerichtet ist und wobei zumindest eine relativ schwere Komponente, wie insbesondere der Atemgasantrieb derart im Gehäuse angeordnet ist, dass bei einer Ausrichtung der Vorrichtung sowohl auf der Bodenwand, als auch auf der als optionale Bodenwand eingerichteten Seitenwand, durch die schwere Komponente ein Schwerpunkt ausgebildet ist.

In einer Weiterbildung ist die Exspirationseinheit aus dem Gehäuse entnehmbar eingerichtet und ausgebildet, wobei die Exspirationseinheit basierend auf einem Klicksystem in das Gehäuse einbringbar und aus dem Gehäuse entnehmbar ist. In der Regel ist die Exspirationseinheit in dem Gehäuse an zumindest einer Seitenwand angeordnet. Die Exspirationseinheit kann eingerichtet sein, mit einem Doppelschlauchsystem verwendbar zu sein.

In Ausgestaltung umfasst die Exspirationseinheit zumindest ein PEEP-Ventil, wobei das PEEP-Ventil einen ersten Abschnitt und einen zweiten Abschnitt umfasst, wobei der erste Abschnitt und der zweite Abschnitt zueinander einen Winkel zwischen 1° und 179°, insbesondere zwischen 42° und 130°, aufweisen. Dies bietet den Vorteil, dass das PEEP-Ventil platzsparend in dem Gehäuse angeordnet werden kann. Insbesondere kann ein derart eingerichtetes PEEP-Ventil derart in dem Gehäuse angeordnet werden, dass der erste Abschnitt an einer ersten Seitenwand angeordnet ist und der zweite Abschnitt an einer zweiten Seitenwand angeordnet ist. Dadurch kann die Exspirationseinheit platzsparend in einer Ecke, zwischen einer ersten Seitenwand und einer zweiten Seitenwand, des Gehäuses angeordnet werden. In der Regel ist das PEEP-Ventil als Schaltventil eingerichtet. Die Exspirationseinheit ist in das Gehäuse der Vorrichtung integriert ausgebildet. Die Exspirationseinheit kann getrennt von dem Pneumatikstrang aus der Vorrichtung entnommen werden.

In weiterer Ausgestaltung umfasst die Vorrichtung einen Schnellwechseladapter, der über ein Drehelement mit dem Trägerrahmen verbunden ist, wobei der Schnellwechseladapter eingerichtet ist, über eine Drehachse des Drehelementes von einer ersten Position in eine zweite Position drehbar zu sein, wobei die zweite Position in einem Winkel zwischen 42° und 180° zu der ersten Position ausgebildet ist. Der Schnellwechseladapter kann optional eingerichtet sein, eine weitere Komponente aufzunehmen.

Der Schnellwechseladapter weist in der Regel eine Aufnahme auf, die eingerichtet ist, eine weitere Komponente aufzunehmen. In der Regel weist die Aufnahme des Schnellwechseladapters ein Rastsystem auf, das eingerichtet ist, über eine Rast- oder Klickverbindung die weitere Komponente aufzunehmen und zu halten. In der Regel weist der Trägerrahmen eine Aufnahme auf, die eingerichtet ist, ein Drehelement aufzunehmen und beweglich zu lagern.

Der Schnellwechseladapter weist in der Regel zumindest ein Drehelement auf, wobei der Schnellwechseladapter über das Drehelement mit dem Trägerrahmen des Pneumatikstrangs beweglich verbunden ist. Insbesondere ist das Drehelement eingerichtet, in die Aufnahme des Trägerrahmens einzugreifen.

Vorrichtung weist beispielsweise eine Aufnahme für einen Schnellwechseladapter auf und umfasst einen Schnellwechseladapter, der über ein Drehelement mit einem Trägerrahmen verbunden ist, wobei der Schnellwechseladapter eingerichtet ist, in der Aufnahme über eine Drehachse des Drehelementes von einer ersten Position in eine zweite Position schwenkbar zu sein, wobei der Schnellwechseladapter in der zweiten Position so aus der Aufnahme herausgeschwenkt ist, dass in der zweiten Position eine Aufnahme für eine O2 Kartusche zugänglich ist und eine O2 Kartusche eingeführt werden kann und wobei der Schnellwechseladapter dann zusammen mit der O2-Kartusche in die erste Position in die Aufnahme zurück geschwenkt werden kann.

In einer Weiterbildung der Ausgestaltung ist der Schnellwechseladapter eingerichtet, eine O2-Kartusche aufzunehmen. Dies bietet den Vorteil, dass die O2-Kartusche bei Verbrauch einfach und schnell über den Schnellwechseladapter aus dem Gehäuse entnehmbar ist. Dies reduziert die Wartungszeit. In der Regel ist die O2-Kartusche im Bereich der Bodenwand der Vorrichtung angeordnet. Dies bietet den Vorteil, dass die O2-Kartusche aus der Vorrichtung entnehmbar ist, ohne das der Pneumatikstrang aus der Vorrichtung entnommen werden muss. O2 steht für Sauerstoff. Optional ist der Schnellwechseladapter eingerichtet, eine andere Kartusche oder eine Vorrichtung zur Medikamentengabe aufzunehmen.

In Weiterbildung weist das Gehäuse eine Aufnahme auf, die eingerichtet ist, die Pneumatikeinheit aufzunehmen, wobei die Pneumatikeinheit in die Aufnahme einbringbar ist. In der Regel weist zumindest eine Seitenwand des Gehäuses eine Aufnahme auf, die eingerichtet ist, den Trägerrahmen aufzunehmen. Die Aufnahme kann eine Einschubvorrichtung sein. Die Pneumatikeinheit wird durch die aufgesetzte Bodenwand gehalten bzw. liegt auf dieser auf. Optional ist der Trägerrahmen über eine Klickverbindung oder über eine Schraubverbindung mit dem Gehäuse verbindbar. Dies bietet den Vorteil, dass der Trägerrahmen starr und fest in dem Gehäuse verankert ist, sodass auch bei einer Positionsänderung der Vorrichtung der Trägerrahmen im Gehäuse unverändert gehalten wird.

Optional weist das Gehäuse zumindest eine Aufnahme auf, die als Führungsschiene ausgebildet ist, in die ein korrespondierend ausgestaltetes viskoelastisches Halteelement des Trägerrahmens eingebracht/eingeschoben werden kann. Dies bietet den Vorteil, dass der Pneumatikstrang mit dem Trägerrahmen in dem Gehäuse auch bei einer Positionsveränderung von der Bodenwand als Standfläche zu der optionalen Bodenwand als Standfläche elastisch in dem Gehäuse gehaltert ist. Alternativ ist zumindest eine Aufnahme als Auflagefläche ausgebildet. Ein viskoelastisches Material ist in der Regel ein Silikon.

Die Vorrichtung ist auch dadurch gekennzeichnet, dass die Vorrichtung eine Benutzerschnittstelle aufweist, die als Bedien-/und Anzeigeelement eingerichtet und ausgebildet ist, wobei das Bedien-/und Anzeigeelement in einer Fläche des Gehäuses der Vorrichtung ausgebildet ist, wobei Bedien-/und Anzeigeelement so großflächig ausgebildet ist, dass es mehr als 45 % oder bevorzugt mehr als 50 % der Fläche des Gehäuses, insbesondere der Dachwand einnimmt.

In einer weiteren Weiterbildung weist die Vorrichtung eine Benutzerschnittstelle auf, die als Bedien-/und Anzeigeelement eingerichtet und ausgebildet ist. In der Regel ist das Bedien-/ und Anzeigeelement als GUI ausgebildet. In der Regel ist das GUI als Touchscreen ausgebildet. Optional umfasst das Bedien-/ und Anzeigeelement haptische Bedienelemente. Ein haptisches Bedienelement kann auf der Dachwand oder an einer Seitenwand der Vorrichtung angeordnet sein. Optional kann das Bedien-/und Anzeigeelement eingerichtet sein, bei einer Einstellung eine akustische oder haptische Bestätigung auszugeben

In Ausgestaltung ist das Bedien-/und Anzeigeelement in einer Fläche des Gehäuses der Vorrichtung, insbesondere der Dachwand, ausgebildet, wobei das Bedien-/und Anzeigeelement eingerichtet ist, eine Darstellung wiederzugeben, wobei die Ausrichtung der Darstellung in Abhängigkeit der gewählten Bodenwand erfolgt. Das Bedien-/ und Anzeigeelement ist somit eingerichtet, eine Orientierung der Ausrichtung der Anzeige zu ändern, wenn die Ausrichtung der Vorrichtung entsprechend einer gewählten Bodenwand geändert wird. Die Vorrichtung ist in der Regel eingerichtet, die Ausrichtung der Anzeige entsprechend der Ausrichtung der Vorrichtung automatisch zu ändern/anzupassen.

In einer Weiterbildung bildet das Bedien-/und Anzeigeelement eine vollständige Fläche des Gehäuses aus, wobei das Bedien-/und Anzeigeelement über Dichtungen mit Seitenwänden des Gehäuses verbunden ist, wobei zumindest eine Dichtung eingerichtet und ausgebildet ist, Schnittstellenelemente und Anschlüsse aufzunehmen. Dies bietet den Vorteil, dass die Schnittstellenelemente und Anschlüsse elastisch und wasserdicht gelagert sind. Optional können die Schnittstellelemente und Anschlüsse eine viskoelastische Kappe aufweisen. Die viskoelatische Kappen kann beispielsweise aus einem Silikon ausgebildet sein.

In einer weiteren Weiterbildung ist das Bedien-/ und Anzeigeelement eingerichtet, eine Umgebungshelligkeit zu erfassen und basierend auf der erfassten Umgebungshelligkeit eine Änderung einer optischen Anzeige des Bedien-/und Anzeigeelementes vorzunehmen, wobei das Bedien-/ und Anzeigeelement eingerichtet ist, bei einer erfassten hellen Umgebungshelligkeit eine Farbintensität zu ändern oder von einer farbigen Anzeige auf eine schwarz-weiße Anzeige zu wechseln. Dies bietet den Vorteil, dass bei einer hellen Umgebungshelligkeit die Sichtbarkeit der Anzeige des Bedien-/und Anzeigeelementes verbessert werden kann.

Das Bedien-/ und Anzeigeelement ist eingerichtet, entsprechend einer erfassten Helligkeit der Umgebungshelligkeit die Farbintensität der Anzeige zu erhöhen oder bei einer starken Helligkeit auf eine schwarz-weiß Anzeige zu wechseln. Durch den Verzicht auf eine farbige Darstellung kann bei einer starken Umgebungshelligkeit durch die Kontrasterhöhung eine bessere Anzeige erzielt werden. Beispielsweise kann das Bedien-/und Anzeigeelement eingerichtet sein, in einem Bereich zwischen 5.000 LUX bis 20.000 LUX, insbesondere zwischen 7.000 LUX und 9.000 LUX von einer farbigen Darstellung auf eine schwarz-weiß Darstellung wechseln. Dies ist insbesondere bei einer mobilen Mitnahme der Vorrichtung, insbesondere im Freien, vorteilhaft. Das Bedien-/ und Anzeigeelement ist eingerichtet, entsprechend einem Akkustand des Akkumulators dimmbar zu sein.

In Ausgestaltung umfasst die Vorrichtung eine digitale Schnittstelle, die eingerichtet ist, erfasste Parameter, Messwerte und Informationen an einen Server oder ein externes Endgerät zu übermitteln und Daten und Informationen über die Schnittstelle zu empfangen. Optional ist die Vorrichtung eingerichtet, die erfassten Werte und/oder Informationen der Messstrecke zu speichern, zu analysieren und/oder zu bewerten. Die Vorrichtung kann über die Schnittstelle mit einem Hustengerät oder einem anderen Beatmungsgerät oder einem Patientenmonitor gekoppelt werden und Daten austauschen.

Optional ist die Vorrichtung eingerichtet, die erfassten, analysierten und/oder bewerteten Messwerte/Parameter an einen externen Server zu übermitteln. Die Übermittlung kann dabei zeitgesteuert, manuell ausgelöst (z.B. am Heimtherapiegeräte oder am Server ausgelöst), ereignisgesteuert (z.B. bei Erkennung bestimmter kritischer Zustände durch das Therapiegerät) oder als dauerhafte Übertragung, zumindest während einer laufenden Therapie, eingerichtet sein.

Eine Übertragung der Messwerte, Parameter und Informationen kann alle 2 Stunden bis 7 Tagen, insbesondere alle 1 bis 3 Tage erfolgen. In einer Ausführung erfolgt die Übertragung zumindest einmal pro Tag/pro 24 Stunden. Optional kann die Schnittstelle eingerichtet sein, Messwerte, Informationen oder Parameter stundenweise zusammenzugefasst zu übertragen oder die Messwerte in Echtzeit zu übertragen. Optional ist durch den Benutzer und/oder durch einen Betreuer ein Übertragungszyklus frei wählbar. Die Schnittstelle des Beatmungsgerätes kann eingerichtet sein, die Übertragung selbsttätig, ggf. wiederholt oder dauerhaft, nach einer oder mehreren fest einprogrammierten und/oder frei eingegebenen Zeitintervallen durchzuführen.

Im Falle eines Ausfalls einer Datenverbindung kann die Speichereinheit der Vorrichtung eingerichtet sein, die Messwerte und/oder die Informationen zumindest einen Tag zu speichern, wobei die Schnittstelle der Vorrichtung eingerichtet ist, die Messwerte an einen externen Server oder ein Endgerät zu übertragen, sobald eine Datenverbindung erneut aufgebaut wurde.

Optional ist die Vorrichtung eingerichtet, über das Bedien- und/ Anzeigeelement Informationen die durch den Benutzer und/oder durch den Betreuer manuell eingegebene Werte mit in die Auswertung von Messwerten einzubeziehen.

In weiterer Ausgestaltung umfasst die Vorrichtung eine Alarmeinheit mit einem Lautsprecher, die eingerichtet ist, bei erkannten Vorkommnissen einen Alarm auszugeben, wobei die Vorrichtung zumindest ein Mikrophon umfasst, dass eingerichtet ist, ein von der Alarmeinheit ausgegebenen Alarm zu überwachen. Dies bietet eine zusätzliche Sicherheitsfunktion für die korrekte Verwendung der Vorrichtung zur Beatmung.

In Ausgestaltung ist die Vorrichtung eingerichtet, mit weiteren Vorrichtungen kombinierbar zu sein. Optional weist die Vorrichtung einen Anschluss für einen Vernebler auf, wobei die Vorrichtung eingerichtet ist, bei einem angeschlossenen Vernebler diesen über die Vorrichtung zu steuern. Die Vorrichtung ist optional eingerichtet, eine Rückmeldung des Verneblers zu erfassen und bei der Steuerung des Verneblers zu berücksichtigen.

Die Vorrichtung umfasst Anschlüsse für einen Server, ein Patientenmanagementsystem, eine Hustenvorrichtung und eine Schlaflaborinfrastruktur. Zudem umfasst die Vorrichtung eine Cloud-Funktion, wobei die Vorrichtung eingerichtet ist, Daten über eine Schnittstelle an eine Cloud zu übermitteln oder einen Anschluss für ein GSM-Modul. In einer Weiterbildung umfasst die Vorrichtung einen Anschluss für ein Krankenschwesterruf-Modul. Zudem umfasst die Vorrichtung zumindest einen SpO2- und/oder einen CO2- Anschluss.

In Ausgestaltung ist der Kühllüfter regelbar eingerichtet, wobei eine Drehzahl des Kühllüfters in Abhängigkeit einer erfassten Temperatur zumindest einer Komponente regelbar ist. Regelbar bedeutet dabei, dass der Kühllüfter ausgeschaltet sein kann oder stufenlos in seiner Drehzahl einstellbar ist. Dies bietet den Vorteil, dass der Kühllüfter durch eine bei einer geringen Wärmeentwicklung geringen Drehzahl eine geringere Geräuschentwicklung aufweist.

In einer Weiterbildung der Ausgestaltung ist die Drehzahl des Kühllüfters basierend auf einer erfassten Temperatur der Steuereinheit, einer erfassten Temperatur des Atemgasantriebs, einer erfassten Temperatur des Akkumulators, einer erfassten Temperatur der Hauptplatine und/oder einem erfassten Ladestrom des Akkumulators regelbar. Optional ist die Drehzahl des Kühllüfters basierend auf einer Kombination zumindest zweiter erfasster Temperaturen von Komponenten regelbar. Die Temperaturen werden in der Regel mittels Temperatursensoren erfasst, die auf der jeweiligen zu messenden Komponente angeordnet sind.

In Ausgestaltung ist die Vorrichtung eingerichtet, einen Ladestrom des Akkumulators zu regeln. Durch die Verringerung des Ladestroms kann eine Erwärmung des Akkumulators verringert werden, wodurch der Kühlaufwand sinkt und damit die Geräuschentwicklung sinkt.

In weiterer Ausgestaltung ist der Ladestrom des Akkumulators in Abhängigkeit einer erfassten Temperatur des Akkumulators, des Kühllüfters oder zumindest einer weiteren Komponente der Vorrichtung regelbar. Die Temperaturen werden in der Regel mittels Temperatursensoren erfasst, die auf der jeweiligen Komponente angeordnet sind. Eine erfasste Temperatur, bei der der Ladestrom heruntergeregelt wird, liegt in der Regel zwischen 30°C und 47°C, insbesondere zwischen 35°C und 42°C.

Des Weiteren betrifft die vorliegende Erfindung einen Schnellwechseladapter für eine Vorrichtung zur Beatmung, umfassend ein Gehäuse umfassend zumindest eine Pneumatikeinheit und zumindest einen Trägerrahmen.

Erfindungsgemäß ist der Schnellwechseladapter über ein Drehelement mit dem Trägerrahmen verbunden, wobei der Schnellwechseladapter eingerichtet ist, über eine Drehachse des Drehelementes von einer ersten Position in eine zweite Position drehbar zu sein, wobei die zweite Position in einem Winkel zwischen 42° und 180° zu der ersten Position ausgebildet ist. Der Schnellwechseladapter weist in der Regel eine Aufnahme auf, die eingerichtet ist, eine weitere Komponente aufzunehmen. In der Regel weist die Aufnahme des Schnellwechseladapters ein Rastsystem auf, das eingerichtet ist, über eine Rast- oder Klickverbindung die weitere Komponente aufzunehmen und zu halten. In der Regel weist der Trägerrahmen eine Aufnahme auf, die eingerichtet ist, ein Drehelement aufzunehmen und beweglich zu lagern. In der Regel ist der Schnellwechseladapter über das Drehelement mit dem Trägerrahmen des Pneumatikstrangs beweglich verbunden. Insbesondere ist das Drehelement eingerichtet, in die Aufnahme des Trägerrahmens einzugreifen.

In Ausgestaltung ist der Schnellwechseladapter eingerichtet, eine O2-Kartusche aufzunehmen.

Dies bietet den Vorteil, dass die O2-Kartusche bei Verbrauch einfach und schnell über den Schnellwechseladapter aus dem Gehäuse entnehmbar ist. Dies reduziert eine Wartungszeit der Vorrichtung. In der Regel ist die O2-Kartusche im Bereich der Bodenwand der Vorrichtung angeordnet. Dies bietet den Vorteil, dass die O2-Kartusche aus der Vorrichtung entnehmbar ist, ohne das der Pneumatikstrang aus der Vorrichtung entnommen werden muss. O2 steht für Sauerstoff. Optional ist der Schnellwechseladapter eingerichtet, eine andere Kartusche oder eine Vorrichtung zur Medikamentengabe aufzunehmen.

Des Weiteren betrifft die vorliegende Erfindung eine Flussmessstrecke für eine Vorrichtung zur Beatmung.

Erfindungsgemäß umfasst die Flussmessstrecke zumindest eine Messeinheit und ist eingerichtet, zumindest einen Parameter eines Atemgases in der Flussmessstrecke zu erfassen, wobei die Flussmessstrecke linear ausgestaltet und eingerichtet ist, wobei an einem Anfang und/oder an einem Ende der Flussmessstrecke ein Gitternetz angeordnet ist und wobei die Flussmessstrecke entnehmbar und aufbereitbar ausgebildet ist.

Im Folgenden werden anhand von stark vereinfachten schematischen Darstellungen bevorzugte Ausführungsbeispiele der Erfindung näher erläutert. Es zeigt:
- Fig. 1: eine perspektivische Draufsicht auf eine Anordnung von Komponenten in einem Gehäuse einer erfindungsgemäßen Vorrichtung zur Beatmung von oben (Dachwand),
- Fig. 2: eine perspektivische Ansicht auf eine Anordnung von Komponenten in dem - in Fig. 1 - gezeigten Gehäuse der erfindungsgemäßen Vorrichtung zur Beatmung von unten (Bodenwand),
- Fig. 3: eine perspektivische Ansicht eines erfindungsgemäßen Atemgasweges A einer Pneumatikeinheit der - in den Fig. 1 und 2 - dargestellten Vorrichtung zur Beatmung,
- Fig. 4: eine perspektivische Ansicht eines erfindungsgemäßen Kühlluftweges B der - in den Fig. 1 und 2 - dargestellten Vorrichtung zur Beatmung,
- Fig. 5: einen Längsschnitt durch eine saugseitige Schalleinheit mit einem Teil des Atemgasweges der erfindungsgemäßen Vorrichtung zur Beatmung,
- Fig. 6: eine Explosionsansicht der - in Fig. 5 gezeigten - saugseitigen Schalleinheit der Vorrichtung zur Beatmung,
- Fig. 7: eine weitere Explosionsansicht der - in den Fig. 5 und 6 - dargestellten Schalleinheit,
- Fig. 8: eine perspektivische Ansicht einer Exspirationseinheit mit einem PEEP-Ventil der - in den Fig. 1 und 2 - dargestellten erfindungsgemäßen Vorrichtung zur Beatmung,
- Fig. 9: eine perspektivische Ansicht einer Pneumatikeinheit der erfindungsgemäßen Vorrichtung zur Beatmung,
- Fig. 10: eine Detailansicht einer Flussmessstrecke der - in Fig. 9 dargestellten - Pneumatikeinheit,
- Fig. 11: eine Detailansicht eines Schnellwechseladapters der erfindungsgemäßen Vorrichtung zur Beatmung,
- Fig. 12: eine Detailansicht einer Benutzerschnittstelle der erfindungsgemäßen Vorrichtung,

In den Figuren weisen dieselben konstruktiven Elemente jeweils dieselben Bezugsziffern auf.

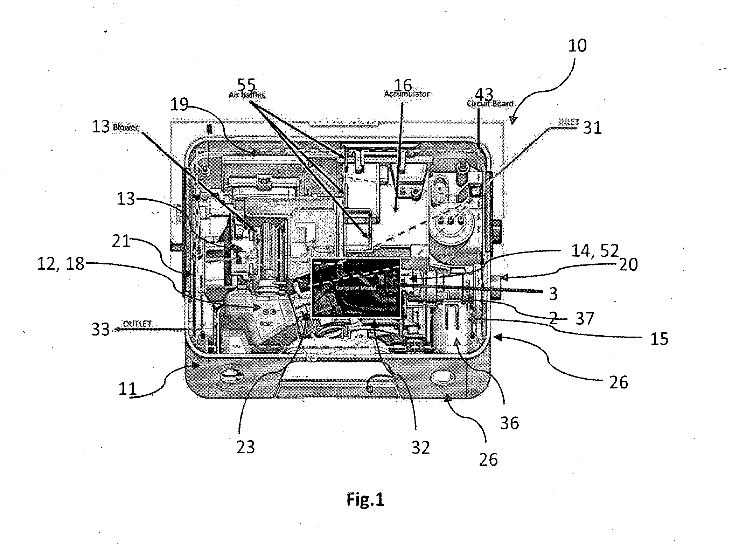

Figur 1 zeigt eine perspektivische Draufsicht auf eine Anordnung von Komponenten in einem Gehäuse 11 einer erfindungsgemäßen Vorrichtung 10 zur Beatmung von oben. Von oben bedeutet dabei eine perspektivische Ansicht über eine geöffnete - nicht gezeigte- Dachwand der Vorrichtung 10. Die Vorrichtung 10 zur Beatmung umfasst zumindest zwei Seitenwände 26 sowie eine Dachwand und eine - ebenfalls nicht dargestellte - Bodenwand. Zumindest eine der Seitenwände 26 kann als optionale Bodenwand eingerichtet sein.

In dem Gehäuse 11 ist eine Pneumatikeinheit 12 dargestellt umfassend einen Trägerrahmen 21 und zumindest eine weitere Komponente. Eine Komponente kann dabei insbesondere ein Atemgasantrieb 13, zumindest eine Messeinheit 14, eine Exspirationseinheit 15, ein Akkumulator 16, eine Steuereinheit 32 und/oder ein Kühllüfter 17 sein. Die Pneumatikeinheit 12 ist als Pneumatikstrang 18 ausgebildet, wobei der Pneumatikstrang 18 von einem Geräteeingang 19 Geräteausgang 20 des Gehäuses 11 ausgebildet ist. Der Pneumatikstrang 18 umfasst den Trägerrahmen 21, auf dem die zumindest eine Komponente angeordnet ist. Der Pneumatikstrang 18 ist aus dem Gehäuse 11 entnehmbar.

Der Pneumatikstrang 18 umfasst zudem zumindest eine Flussmessstrecke 23, wobei zumindest ein Sensor 52 in der Flussmessstrecke 23 angeordnet ist und eingerichtet ist, zumindest einen Wert/Parameter der durch den Atemgasantrieb 13 durch den Pneumatikstrang 18 geführten Atemluft zu erfassen. Aus dem Pneumatikstrang 18 sind in aus dem Gehäuse 11 ausgebautem Zustand Komponenten entnehmbar.

Dargestellt ist zudem eine Exspirationseinheit 15. Die Exspirationseinheit 15 ist in der vorliegenden Ausführungsform in einem Eckbereich des Gehäuses 11 angeordnet. Die Expeditionseinheit 15 kann in einem Seitenbereich/an einer der Seitenwände 26 des Gehäuses 11 angeordnet sein. Die Exspirationseinheit 15 ist aus dem Gehäuse 11 entnehmbar, wobei die Exspirationseinheit 15 basierend auf einem Klicksystem in das Gehäuse 11 einbringbar und aus dem Gehäuse 11 entnehmbar ist. Die Exspirationseinheit 15 umfasst ein PEEP-Ventil, wobei das PEEP-Ventil einen ersten Abschnitt 36 und einen zweiten Abschnitt 37 umfasst, wobei der erste Abschnitt 36 und der zweite Abschnitt 37 zueinander einen Winkel zwischen 1° und 179°, insbesondere zwischen 42° und 130°, aufweisen.

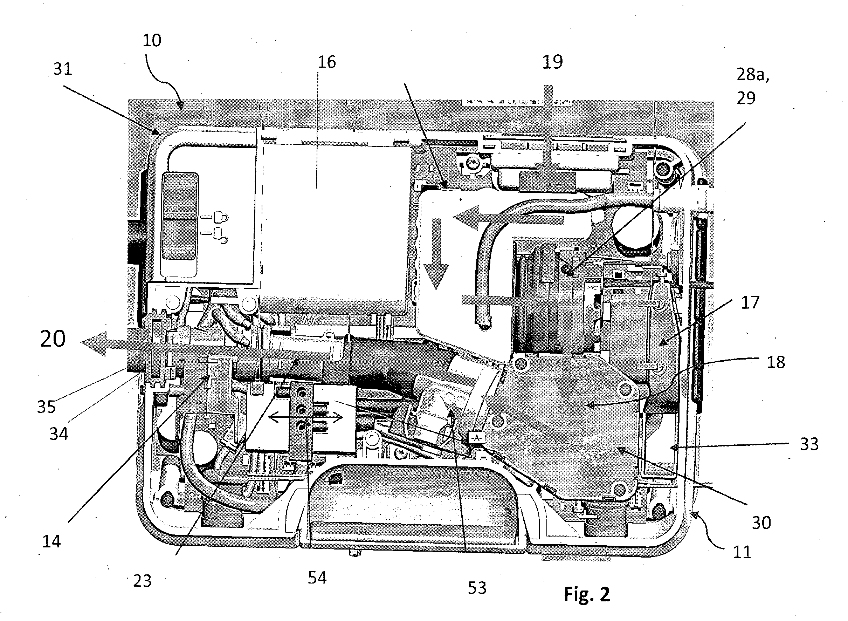

Figur 2 zeigt eine perspektivische Ansicht auf eine Anordnung von Komponenten in dem - in Fig. 1 - gezeigten Gehäuse der erfindungsgemäßen Vorrichtung 10 zur Beatmung von unten. Von unten bedeutet dabei eine Ansicht auf eine (geöffnete) Bodenwand der Vorrichtung 10. In der Regel ist die Bodenwand abnehmbar eingerichtet, sodass über die geöffnete Bodenwand der - in Fig. 1 beschriebene - Pneumatikstrang 18 aus dem Gehäuse 11 entnehmbar ist. Der Pneumatikstrang 18 ist in dem Gehäuse 11 der Vorrichtung 10 von zumindest einer - in Fig. 2 gezeigtenviskoelastischen oder elastomeren Aufhängung 34 gehaltert. Ein viskoelastisches Material kann ein Silikon sein. Der Pneumatikstrang 18 ist im Kontaktbereich des Pneumatikstrangs 18 zum Gehäuse 11, insbesondere im Bereich des Atemluftausgangs 35, des Kühllufteingangs 31 und des Kühllüfters 17, gehaltert.

Der Pneumatikstrang 18 weist eine Saugseite 29 und eine Druckseite 30 auf, wobei der Pneumatikstrang 18 zudem zumindest eine saugseitige Schalleinheit 28a, 28b sowie eine druckseitige Schalleinheit 28c umfasst. Eine saugseitige Schalleinheit 28b kann beispielsweise direkt am Geräteeingang 19 ausgebildet sein. Beispielsweise kann eine derartige - nicht gezeigte - Schalleinheit 28b als Luftumlenkung ausgestaltet sein.

In der Figur 2 sind zudem ein Rückschlagventil mit Bypass 53, ein Druckaufbauventil 54, eine Flussmessstrecke 23 und ein Schnellwechseladapter 14 dargestellt. Auch dargestellt ist der Akkumulator 16.

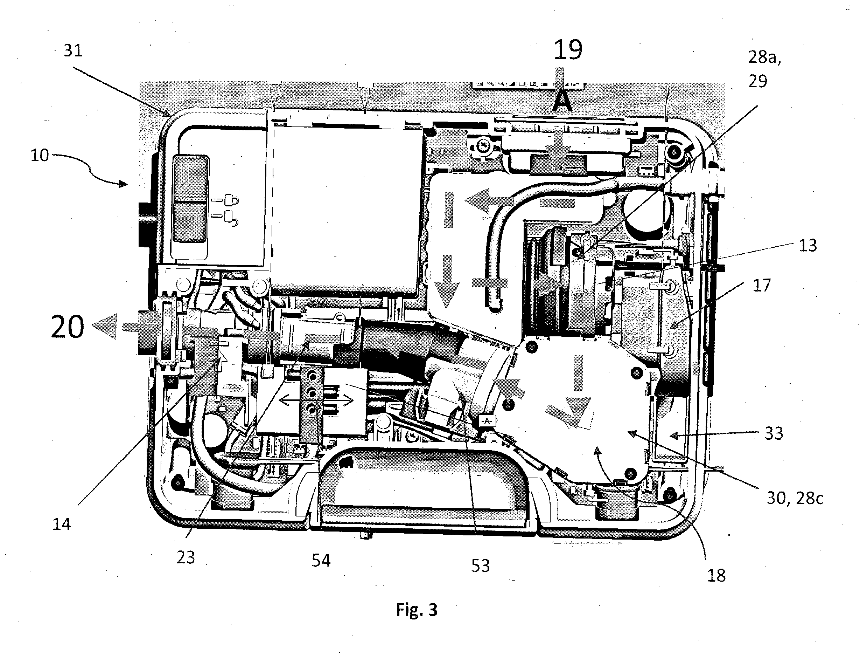

Figur 3 zeigt eine perspektivische Ansicht eines erfindungsgemäßen Atemgasweges A eines Pneumatikstrangs 18 der - in den Fig. 1 und 2 - dargestellten - Vorrichtung 10 zur Beatmung. Der Atemgasweg A erstreckt sich von einem Geräteeingang 19 zu einem Geräteausgang 20 der Vorrichtung 10. Der Atemgasweg A umfasst den Pneumatikstrang 18 mit dem Atemgasantrieb 13 und den Schalleinheiten 28a und 28c. Zudem umfasst der Atemgasweg die Flussmessstrecke 23 und zumindest einen Sensor. Der Atemgasantrieb 13 erzeugt einen Sog, sodass über den Geräteeingang 19 Umgebungsluft in die Vorrichtung 10 eingezogen wird. Der Atemgasweg A ist eingerichtet, Atemluft von der Umgebung zu einem Patienten zu führen. Die Schalleinheiten 28a und 28c ermöglichen eine Geräuschreduzierung der Luftführung.

In einer weiteren - nicht gezeigten Ausführungsform - umfasst der Atemgasweg A eine weitere Luftumlenkvorrichtung 28b, die eingerichtet ist, die mit dem Atemgasantrieb eingezogene Luft entgegen der Luftstromsogrichtung in die Vorrichtung 10 einzuführen.

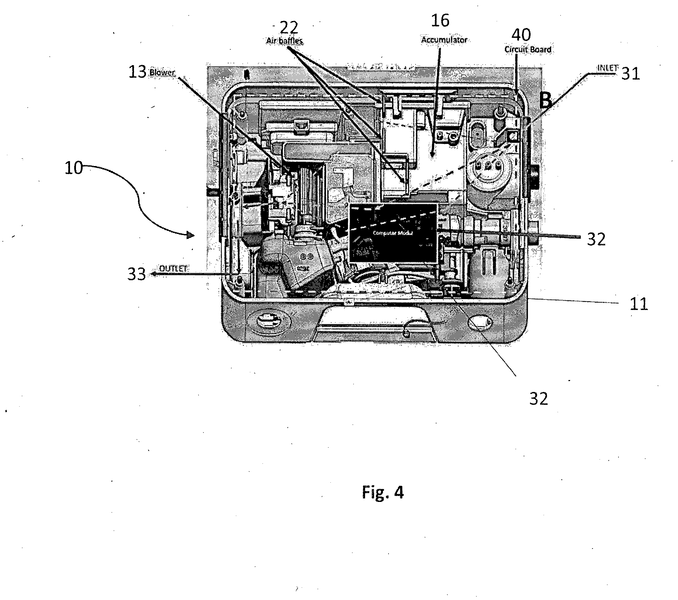

Figur 4 zeigt eine perspektivische Ansicht eines erfindungsgemäßen Kühlluftweges B der - in den Fig. 1 und 2 - dargestellten Vorrichtung 10 zur Beatmung. Der Kühlluftweg B erstreckt sich von einem Kühllufteingang 31 zu einem Kühlluftausgang 33. Dabei wird durch einen Kühllüfter 17, der am Kühlluftausgang 33 angeordnet ist, Kühlluft/Umgebungsluft zur Kühlung in die Vorrichtung 10 gesogen.

Die Kühlluft wird über Luftleitbleche 22 in dem Gehäuse 11 geführt/kanalisiert. Dabei sind die Luftleitbleche 22 derart angeordnet, dass die Kühlluft in dem Gehäuse 11 über zumindest den Atemgasantrieb 13, die Steuereinheit 32 und die Hauptplatine 40 geführt ist. Dabei ist die Komponente, die die höchste Wärmeentwicklung im Betrieb aufweist, in Kühlluftwegstromrichtung am Ende des Kühlluftweges B angeordnet. Dadurch wird die Wärme der Komponenten mit der höchsten Wärmeentwicklung nur eine geringe Strecke im Kühlluftweg mitgeführt, bevor sie aus dem Gehäuse 11 ausgeleitet wird. Dadurch wird die Wärme der Steuereinheit 32, des Atemgasantriebs 13 und der Hauptplatine 40 gezielt kurzfristig in dem Kühlluftweg B geführt und schnellstmöglich ausgeleitet.

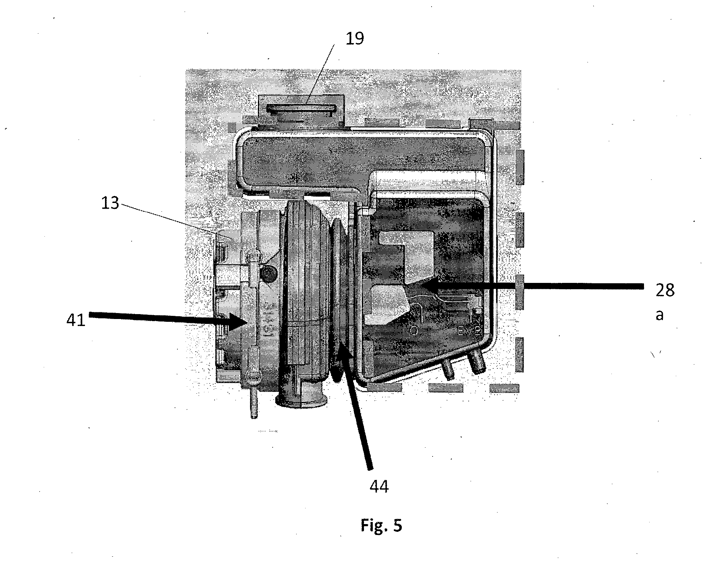

Figur 5 zeigt einen Längsschnitt durch eine saugseitige Schalleinheit 28a mit einem Teil des Atemgasweges A der erfindungsgemäßen Vorrichtung zur Beatmung. Die saugseitige Schalleinheit 28a ist dem Geräteeingang 19 in Luftströmungsrichtung nachgeschaltet angeordnet. Dargestellt ist der Geräteeingang 19, eine Entkopplungsvorrichtung 44 sowie eine Gebläsekappe 41. Die saugseitige Schalleinheit 28a ist eingerichtet, einen Geräuschpegel der durch den Atemgasweg B geführten Atemluft zu reduzieren. Die Gebläsekappe 41 ist eingerichtet, an den Atemgasantrieb 13 anfügbar zu sein. Somit ist die saugseitige Schalleinheit 28a mit dem Atemgasantrieb 13 modular zusammenfügbar.

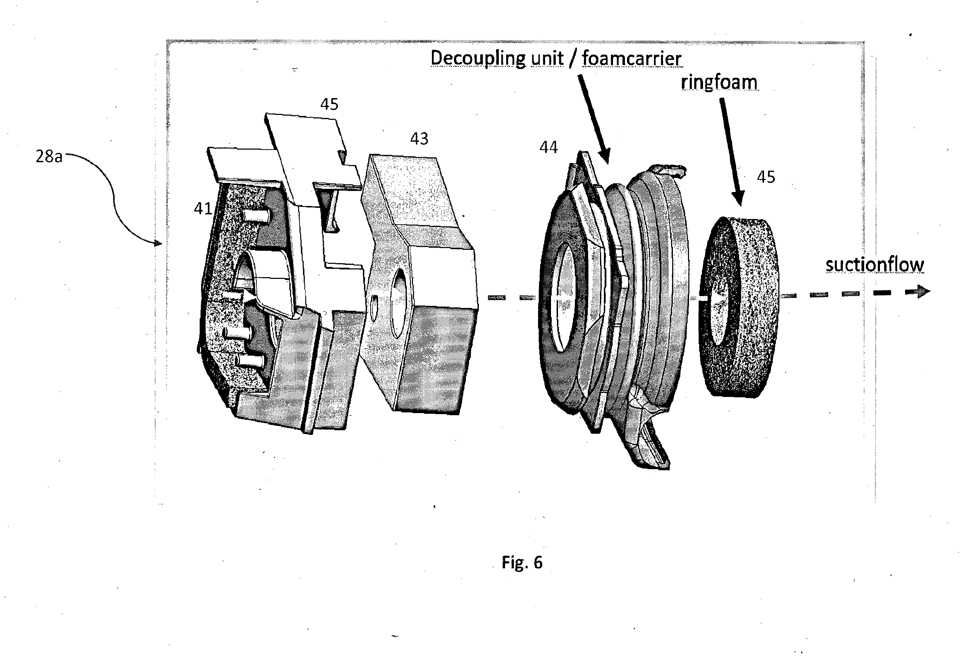

Figur 6 zeigt eine Explosionsansicht der - in Fig. 5 gezeigten - saugseitigen Schalleinheit 28a der Vorrichtung 10 zur Beatmung. Dargestellt ist - in Fig. 5 gezeigte - Gebläsekappe 41, ein Schaumhalter 42, ein Absorberschaum 43, die Entkopplungsvorrichtung 44 und ein Ringschaum 45. Der Schaumhalter 42 ist eingerichtet, den Absorberschaum 43 aufzunehmen und zu halten. Die Entkopplungsvorrichtung 44 ist in der Regel aus einem viskoelastischen Material ausgebildet, insbesondere aus einem Silikon.

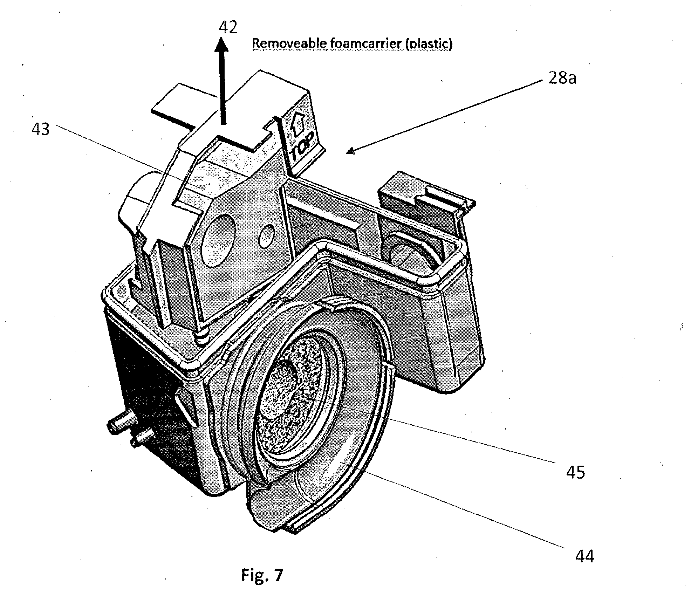

Figur 7 zeigt eine weitere Explosionsansicht der - in den Fig. 5 und 6 - dargestellten Schalleinheit 28a. Dabei ist dargestellt, wie der Schaumhalter 42 mit dem Absorberschaum 43 aus der Schalleinheit 28a entnehmbar ist. Dies ist besonders vorteilhaft, da der Schaumhalter 42 mit dem Absorberschaum 43 einzeln aus der Schalleinheit entnehmbar und aufbereitbar ist.

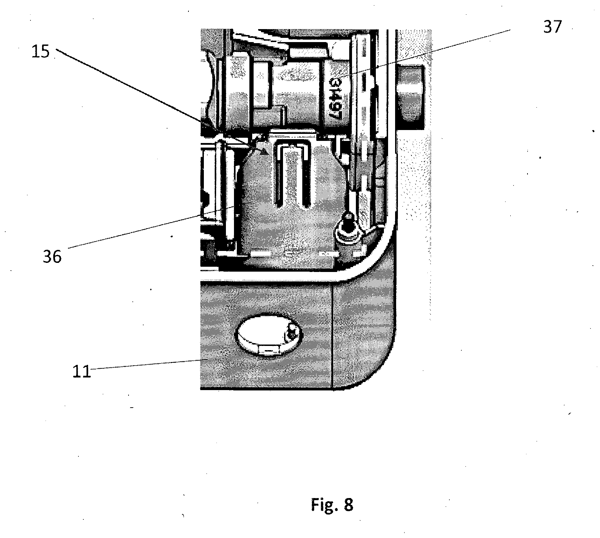

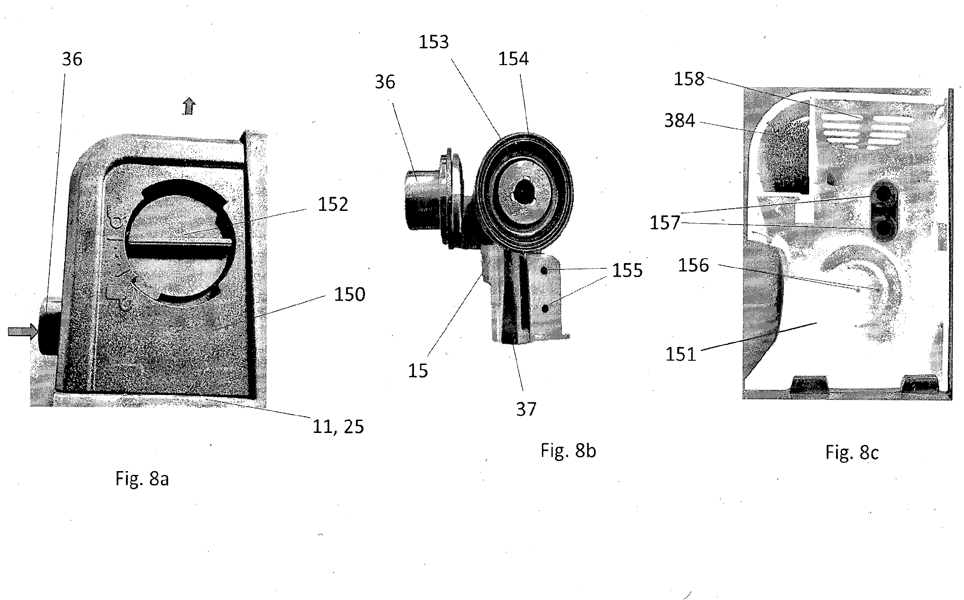

Figur 8 zeigt eine perspektivische Ansicht einer Exspirationseinheit 15 mit einem PEEP-Ventil der - in den Fig. 1 und 2 - dargestellten erfindungsgemäßen Vorrichtung 10 zur Beatmung. Die Exspirationseinheit 15 ist aus dem Gehäuse 11 entnehmbar eingerichtet und ausgebildet, wobei die Exspirationseinheit 15 basierend auf beispielsweise einem Klicksystem in das Gehäuse 11 eingesetzt wird. Durch das Klicksystem kann die Exspirationseinheit 15 besonders schnell aus dem Gehäuse 11 entnommen werden und aufbereitet oder erneuert werden. In der vorliegenden Ausführungsform ist die Exspirationseinheit 15 rechtwinkling ausgestaltet, d.h. ein erster Abschnitt 36 der Exspirationseinheit 15 und ein zweiter Abschnitt 37 der Exspirationseinheit 15 weisen zueinander einen Winkel zwischen 1° und 179°, insbesondere zwischen 42° und 130°, auf. In der Regel ist das PEEP-Ventil als Schaltventil eingerichtet. Der Eingang der Exspirationseinheit ist in der Regel vor der Flussmessstrecke ausgebildet.

Figur 8a zeigt die Unterseite 25 des Gehäuses 11 der Vorrichtung 10 zur Beatmung. Ein Deckel 150 verschließt die Aufnahme 151 für die Exspirationseinheit 15. Der Deckel weist einen Schnellverschluss 152 auf, der beispielsweise wie ein Drehschloss funktionniert. Ein erster Abschnitt 36 der Exspirationseinheit dient als Stutzen für den Exspirationssschlauch, über den Ausatemluft (Pfeil) zu der Exspirationseinheit geführt wird. Nach Passage der Exspirationseinheit (mit Flussmessstrecke) verlässt die Ausatemluft die Exspirationseinheit (in einem Winkel von etwa 90°) über den zweiten Abschnitt 37.

Fig. 8b zeigt die Exspirationseinheit 15 mit dem PEEP-Ventil 153, welches den Atemgasfluss über die Exspirationseinheit 15 freigeben oder sperren kann. Das PEEP-Ventil 153 weist dazu eine Membran 154 auf, die mit einem Steuerdruck beaufschlagt werden kann, um Atemgasfluss über die Exspirationseinheit 15 zu sperren oder freizugeben. An dem zweiten Abschnitt 37 befinden sich zwei Abgriffe 155 für die Flussmessung.

Figur 8c zeigt die Aufnahme 151 für die Exspirationseinheit 15. Hier befindet sich eine Aufnahme für die Membran 154, wobei in der Aufnahme zwei Passagen 156 für den Steuerdruck angeordnet sind. Wenn die Exspirationseinheit 15 in der Aufnahme ist, kann der Steuerdruck (über das Gebläse erzeugt) über die Passagen 156 an die Membran geleitet werden. Die Aufnahme 151 weist zudem zwei Flusspassagen 157 auf, über die der Fluss an Exspirationsgas, der durch die Exspirationseinheit 15 fließt, gemessen werden kann. Der Flusssensor liegt innerhalb des Gehäuses. Die Flusspassagen 157 sind so angeordnet, dass die Abgriffe 155 der Exspirationseinheit 15 unmittelbar über den Flusspassagen 157 liegen (wenn die Exspirationseinheit 15 in der Aufnahme ist) und eine im Wesentlichen leckagefreie Verbindung von der Exspirationseinheit 15 zu dem Flusssensor sichergestellt ist. Der der Fluss an Exspirationsgas verlässt dir Aufnahme durch die Exspirationsöffnung 158. Beispielsweise ist die Exspirationsöffnung 158 abgewandt von dem ersten Abschnitt 36 und abgewandt von dem Geräteeingang 19 angeordnet.

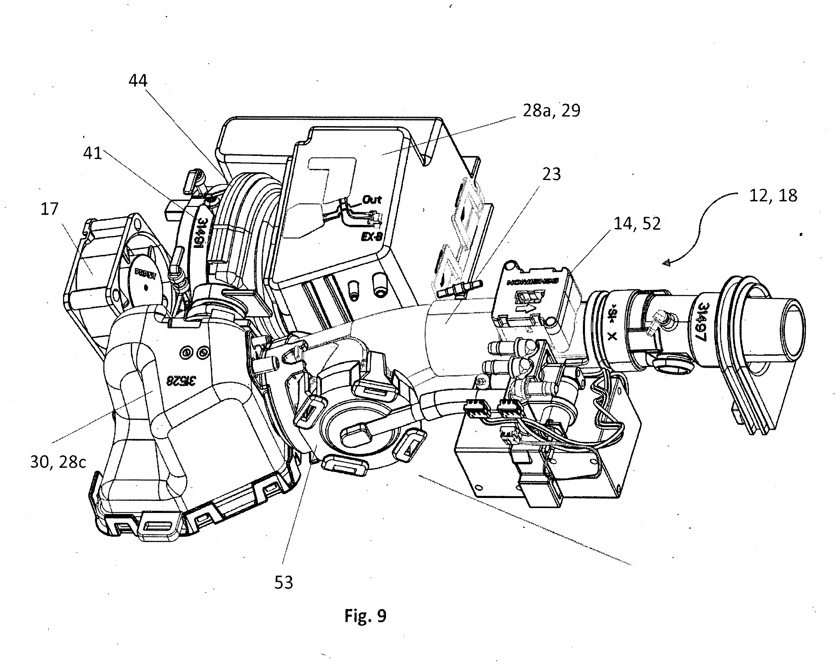

Figur 9 zeigt eine perspektivische Ansicht einer Pneumatikeinheit 12 der erfindungsgemäßen Vorrichtung zur Beatmung. Die Pneumatikeinheit 12 ist in der vorliegenden Ausführungsform als Pneumatikstrang 18 ausgestaltet. Es sind auch andere zusammenhängende Anordnungsweisen der Komponenten zu einer Pneumatikeinheit 12 denkbar. Der Pneumatikstrang 18 ist von dem - nicht gezeigten - Geräteeingang zu dem Geräteausgang des Gehäuses ausgebildet. Der Pneumatikstrang 18 umfasst den - nicht gezeigten -Trägerrahmen 21 auf dem die zumindest eine weitere Komponente angeordnet ist. Die zumindest eine weitere Komponente kann dabei insbesondere der Atemgasantrieb, die zumindest eine Messeinheit 14, 52, die Exspirationseinheit, der Akkumulator, die Steuereinheit und/oder der Kühllüfter 17 sein. Der Pneumatikstrang 18 ist einteilig umfassend alle auf dem Trägerrahmen angeordneten Komponenten aus dem Gehäuse 11 entnehmbar. Der Pneumatikstrang 18 weist die Saugseite 29 und die Druckseite 30 auf, wobei der Pneumatikstrang 18 zudem zumindest die eine saugseitige Schalleinheit 28a sowie die eine druckseitige Schalleinheit 28c umfasst.

Der Pneumatikstrang 18 umfasst als weitere Komponente die Flussmessstrecke 23, wobei der zumindest eine Sensor 52 in der Flussmessstrecke 23 angeordnet ist und zumindest einen Wert/Parameter der durch den Atemgasantrieb 13 durch den Pneumatikstrang 18 geführten Atemluft erfasst.

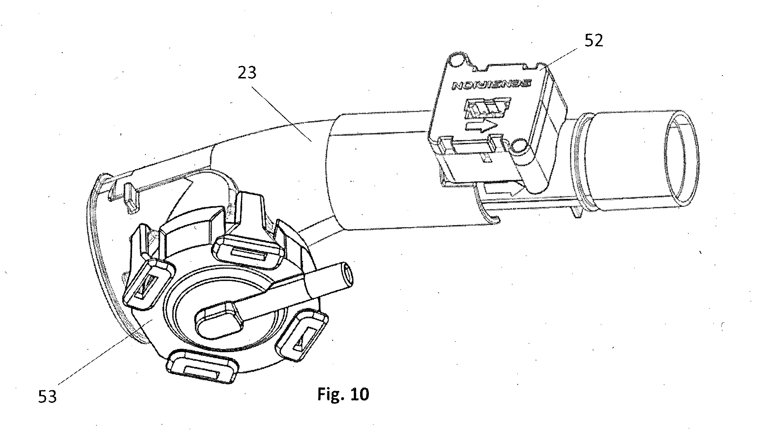

Figur 10 zeigt eine Detailansicht einer Flussmessstrecke 23 der - in Fig. 9 - dargestellten Pneumatikeinheit 12. Die Flussmessstrecke 23 ist eingerichtet, zumindest einen Wert/Parameter der durch den Atemgasantrieb durch den Pneumatikstrang geführten Atemluft zu erfassen. In der Regel umfasst die Flussmessstrecke 23 zumindest einen Sensor 52, der eingerichtet ist, einen Druck- und/oder Fluss der Atemluft in dem Atemgasweg B zu erfassen. Die Vorrichtung ist eingerichtet, die erfassten Werte/Parameter zu speichern. Optional ist die Vorrichtung eingerichtet, die erfassten Werte/Parameter zu analysieren und/oder über eine Schnittstelle an einen Server und/oder ein Endgerät zu übermitteln. Zudem ist in der Figur 10 der Bypass 53 des Rückschlagventils dargestellt.

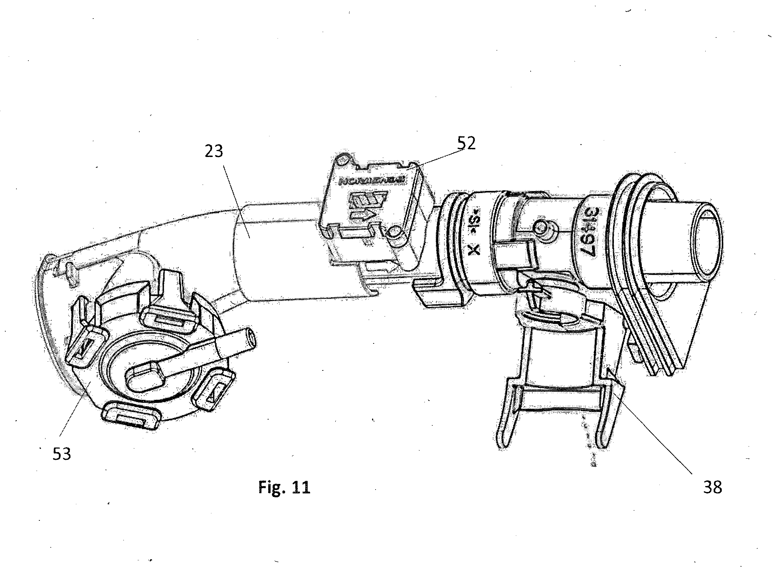

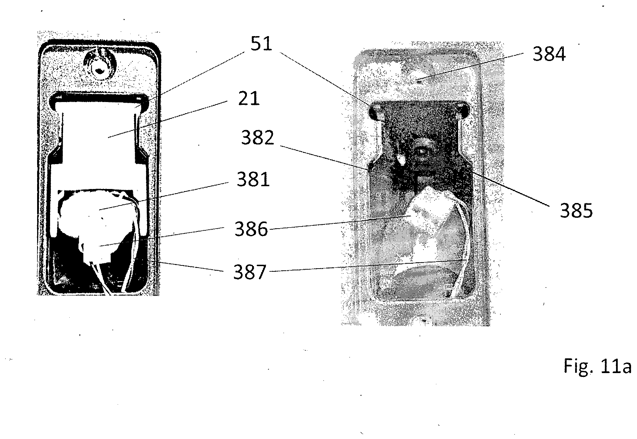

Figur 11 zeigt eine Detailansicht eines Schnellwechseladapters 38 der erfindungsgemäßen Vorrichtung zur Beatmung.

Der Schnellwechseladapter 38 ist über ein Drehelement mit dem Trägerrahmen verbunden, wobei der Schnellwechseladapter 38 eingerichtet ist, über eine Drehachse des Drehelementes von einer ersten Position in eine zweite Position drehbar zu sein. Die zweite Position ist vorteilhafterweise in einem Winkel zwischen 42° und 180° zu der ersten Position ausgebildet. Der Schnellwechseladapter 38 kann eine weitere Komponente, insbesondere eine O2-Kartusche aufnehmen. Hierfür weist der Schnellwechseladapter 38 eine Aufnahme auf, die in der Regel korrespondierend zu der aufzunehmenden Komponente ausgebildet und eingerichtet ist. Zur Aufnahme weist die Aufnahme eine Rast-/Klicksystem auf. In der Figur 11 ebenfalls dargestellt sind der Bypass 53 des Rückschlagventils, die Flussmessstrecke 23 sowie der Sensor 52.

Fig. 11a zeigt den Schnellwechseladapter (38) für eine O2-Kartusche (381).

Der Schnellwechseladapter (38) kann in eine Aufnahme (382) für den Schnellwechseladapter eingesetzt werden. Die Aufnahme befindet sich in dem Gehäuse (11) der Vorrichtung, bevorzugt in der Bodenwand (25). Die Bodenwand weist einen abnehmbaren Deckel für die Aufnahme (382) auf, der beispielsweise mit Schrauben oder Rastelementen (384) gehalten wird. Der Deckel ist hier nicht dargestellt. Die Aufnahme (382) weist einen Zugang (385) zu dem Atemgasstrom auf. Der Zugang stellt die pneumatische Verbindung für die O2-Kartusche (381) dar.

Der Schnellwechseladapter (38) ist über ein Drehelement (51) mit dem Trägerrahmen (21) verbunden. Der Trägerrahmen (21) weist eine Aufnahme (383) für eine O2-Kartusche auf. Die Aufnahme (383) ist so ausgebildet, dass die O2 Kartusche eingeführt wird und dort gehalten wird. Die Aufnahme (383) kann ein Schraubgewinde aufweisen, wobei die O2-Kartusche ein korrespondierendes Gegenstück aufweist.

Der Schnellwechseladapter (38) ist eingerichtet, in der Aufnahme (382) über eine Drehachse des Drehelementes (51) von einer ersten Position in eine zweite Position drehbar zu sein. Die zweite Position ist in einem Winkel zwischen 42° und 180° zu der ersten Position ausgebildet. In der zweiten Position ist der Schnellwechseladapter (38) aus der Aufnahme (382) für den Schnellwechseladapter herausgeschwenkt. In der zweiten Position ist die Aufnahme (383) zugänglich, so dass die O2 Kartusche eingeführt werden kann. Zusammen mit der O2-Kartusche kann der Schnellwechseladapter (38) in die erste Position in die Aufnahme (382) geschwenkt werden. In der ersten Position verrastet der Schnellwechseladapter (38) und die O2 Kartusche wird pneumatisch, über einen Zugang (385) mit dem Gasfluss verbunden, um dort wird den Sauerstoffgehalt (FiO2) zu bestimmen. Die O2-Kartusche (381) weist ein erstes Ende auf, welches pneumatisch mit dem Atemgasstrom kommuniziert und ein zweites Ende auf, welches elektrisch über einen Stecker (386) konnektiert wird. In der Aufnahme (382) ist daher ein Stecker (386) mit einem Kabel (387) angeordnet, um mit der Kartusche verbunden zu werden.

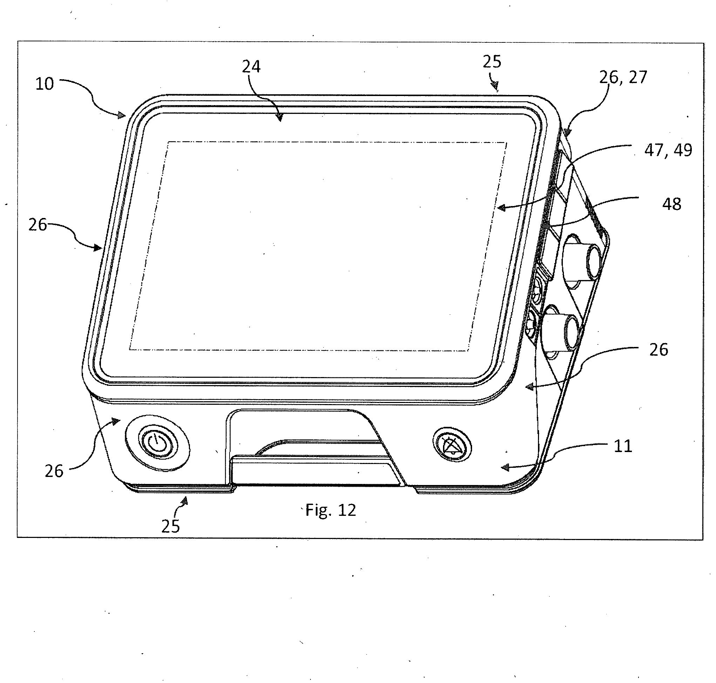

Figur 12 zeigt eine Detailansicht einer Benutzerschnittstelle 47 der erfindungsgemäßen Vorrichtung 10. Die Vorrichtung 10 umfasst die Benutzerschnittstelle 47, die als GUI ausgebildet ist. Die Benutzerschnittstelle 47 ist flächig in der Dachwand 24 des Gehäuses 11 angeordnet. Die Diagonale (oder die Länge der Diagonalen) der Benutzerschnittstelle 47 beträgt größer als 70%, bevorzugt größer 75% der Diagonalen der Gehäusefläche, in der die Benutzerschnittstelle 47 angeordnet ist.

Die Benutzerschnittstelle 47 ist eingerichtet, eine Anzeige/Darstellung entsprechend einer gewählten Bodenwand 25 darzustellen. Sowohl die Bodenwand 25 als auch eine Seitenwand 26, die als optionale Bodenwand 25 eingerichtet ist, können somit die Ausrichtung der Anzeige/Darstellung der Vorrichtung 10 bestimmen. Entsprechend der jeweils gewählten Bodenwand 25 passt die Vorrichtung 10 die Ausrichtung der Anzeige/Darstellung automatisch an. Das GUI ist in der Regel ein Touchscreen. Optional kann die Vorrichtung haptische Bedienelemente umfassen. Beispielsweise können haptische Drehelemente an zumindest einer der Seitenwände 26 angeordnet sein.

## Patentansprüche

1. Vorrichtung (10) zur Beatmung umfassend ein Gehäuse (11), eine Benutzerschnittstelle (47) und eine Pneumatikeinheit (12), wobei die Pneumatikeinheit (12) zumindest je eine der Komponenten Atemgasantrieb (13), Messeinheit (14), Steuereinheit (32) und Schalleinheit (22) umfasst wobei die Pneumatikeinheit (12) als Pneumatikstrang (18) ausgebildet ist, wobei der Pneumatikstrang (18) einen Atemgasweg (B) von einem Geräteeingang (19) zu einem Geräteausgang (20) des Gehäuses (11) ausbildet und wobei zumindest eine Komponente der Pneumatikeinheit (12) oder der Pneumatikstrang (18) aus dem Gehäuse (11) entnehmbar ist,
wobei
ein Kühlluftweg, angetrieben von einem Kühllüfter (17), von einem Kühllufteingang (31) zu einem Kühlluftausgang (33) durch das Gehäuse (11) geführt ist, wobei zumindest die Steuereinheit (32), der Atemgasantrieb (13) und eine Hauptplatine (40) in dem Kühlluftweg angeordnet sind,
**dadurch gekennzeichnet, dass** die Steuereinheit (32), der Atemgasantrieb (13) und die Hauptplatine (40) derart in dem Kühlluftweg angeordnet sind, dass die Komponente, die die höchste Wärmeentwicklung im Betrieb aufweist, in Kühlluftwegstromrichtung am Ende des Kühlluftweges angeordnet ist, und die Komponente, die die höchste Wärmeentwicklung im Betrieb aufweist der Atemgasantrieb ist.

2. Vorrichtung (10) zur Beatmung nach Anspruch 2, **dadurch gekennzeichnet,**
**dass** der Pneumatikstrang (18) von dem Geräteeingang (19) zu dem Geräteausgang (20) des Gehäuses (11) ausgebildet und angeordnet ist und einen Trägerrahmen (21) umfasst, auf dem zumindest eine Komponente, insbesondere der Atemgasantrieb (13), zumindest die Schalleinheit (22),
zumindest eine Flussmessstrecke (23), zumindest die Messeinheit (14) und der Kühllüfter (17) angeordnet sind, wobei der Pneumatikstrang (18) aus dem Gehäuse (11) entnehmbar ist, wobei der Pneumatikstrang (18) in dem Gehäuse (11) der Vorrichtung (10) von zumindest einer viskoelastischen oder elastomeren Aufhängung (34) gehaltert ist.

3. Vorrichtung (10) nach einem der voranstehenden Ansprüche, **dadurch gekennzeichnet dass** der Atemgasweg (B) von dem Kühluftweg (A) pneumatisch getrennt ist und/oder dass der Geräteeingang (19) in dem Gehäuse (11) derart beabstandet von dem Kühlluftausgang (33) angeordnet ist, dass durch den Geräteeingang (19) keine Luft aus dem Kühlluftausgang (33) angesaugt wird.

4. Vorrichtung (10) nach Anspruch 1, **dadurch gekennzeichnet, dass** das Gehäuse (11) eine Dachwand (24), zumindest eine Bodenwand (25) und zumindest zwei Seitenwände (26) aufweist, wobei der Geräteeingang (19) und der Geräteausgang (20) an zwei sich nicht gegenüberliegenden Seitenwänden (26) des Gehäuses (11) ausgebildet sind.

5. Vorrichtung (10) nach einem der voranstehenden Ansprüche, **dadurch gekennzeichnet, dass** zumindest eine Seitenwand (26) als optionale Bodenwand (27) eingerichtet ist.

6. Vorrichtung (10) nach Anspruch 5 **dadurch gekennzeichnet, dass** die Bodenwand (27) des Gehäuses (11) abnehmbar ist und der Pneumatikstrang (18) über eine Öffnung der Bodenwand (27) einteilig entnehmbar ist.

7. Vorrichtung (10) nach einem der Ansprüche 5 oder 6, **dadurch gekennzeichnet, dass** ein Akkumulator (16) und der Atemgasantrieb (13) derart im Gehäuse (11) angeordnet sind, dass bei einer Ausrichtung der Vorrichtung (10) sowohl auf der Bodenwand (25) als auch auf der als optionale Bodenwand (27) eingerichteten Seitenwand (26) durch den Akkumulator (16) und den Atemgasantrieb (13) ein Schwerpunkt ausgebildet ist.

8. Vorrichtung (10) nach einem der Ansprüche 5 bis 7, **dadurch gekennzeichnet, dass** sowohl in der Bodenwand (25), als auch in der als optionale Bodenwand (27) eingerichteten Seitenwand (26), kein Kühllufteingang (31) oder Kühlluftausgang (33) oder Geräteeingang (19) oder Geräteausgang (20) angeordnet ist.

9. Vorrichtung (10) nach einem der voranstehenden Ansprüche, **dadurch gekennzeichnet, dass** der Pneumatikstrang (18) zumindest zwei Schalleinheiten (28a, 28b, 28c) aufweist, wobei zumindest eine Schalleinheit (28a, 28b) auf einer Saugseite (29) des Pneumatikstrangs (18) und eine Schalleinheit (28c) auf einer Druckseite (30) des Pneumatikstrangs (18) angeordnet ist, wobei die Schalleinheit (22) auf der Saugseite (29) an einer Aufnahme des Atemgasantriebs (13) angeordnet ist und eine Gebläsekappe (41), einen Schaumhalter (42), zumindest einen Absorberschaum (43), eine Entkopplungsvorrichtung (44) und zumindest einen Ringschaum (45) umfasst.

10. Vorrichtung (10) nach einem der voranstehenden Ansprüche, **dadurch gekennzeichnet, dass** die Vorrichtung (10) eine Kühleinheit (39) aufweist, wobei die Kühleinheit (39) den Kühllufteingang (31) und den Kühlluftausgang (33) aufweist, wobei der Kühllüfter (17) derart in dem Gehäuse (11) angeordnet ist, das ein Luftstrom von dem Kühllufteingang (31) zumindest über die Steuereinheit (32) und den Atemgasantrieb (13) in Richtung des Kühlluftausgangs (33) geführt ist.

11. Vorrichtung (10) nach einem der voranstehenden Ansprüche, **dadurch gekennzeichnet, dass** der Kühlluftweg (A) gegenläufig und unabhängig zu dem Atemgasweg des Pneumatikstrangs (18) ausgebildet und eingerichtet ist.

12. Vorrichtung (10) nach einem der voranstehenden Ansprüche, **dadurch gekennzeichnet, dass** die Pneumatikeinheit (12) eine Flussmesstrecke (23) umfasst, die zumindest eine Messeinheit (14) umfasst und eingerichtet ist, zumindest einen Parameter eines Atemgases in der Flussmessstrecke (23) zu erfassen, wobei die Flussmessstrecke (23) linear ausgestaltet und eingerichtet ist, wobei an einem Anfang und/oder an einem Ende der Flussmessstrecke (23) ein Gitternetz angeordnet ist und wobei die Flussmesstrecke (23) entnehmbar und aufbereitbar ausgebildet ist.

13. Vorrichtung (10) nach Anspruch 12, **dadurch gekennzeichnet, dass** die mindestens eine Messeinheit (14) aus dem Gehäuse (11) entnehmbar ausgebildet und eingerichtet ist, wobei die mindestens eine Messeinheit (14) zumindest einen Sensor (52) zur Erfassung zumindest eines Parameters der über den Atemgasweg geführten Atemluft umfasst.

14. Vorrichtung (10) nach einem der voranstehenden Ansprüche, **dadurch gekennzeichnet, dass** eine Exspirationseinheit (15) aus einer Aufnahme (151) des Gehäuses (11) entnehmbar ausgebildet ist, wobei die Exspirationseinheit (15) zumindest ein PEEP-Ventil umfasst, welches eine Membran (154) aufweist, die mit einem Steuerdruck beaufschlagt werden kann, um einen Atemgasfluss über die Exspirationseinheit zu sperren oder freizugeben und/oder wobei die die Exspirationseinheit (15) zumindest einen Abgriff (155) für eine Flussmessung aufweist.

15. Vorrichtung (10) nach einem der voranstehenden Ansprüche, **dadurch gekennzeichnet, dass** die Vorrichtung (10) eine Aufnahme (382) für einen Schnellwechseladapter aufweist und einen Schnellwechseladapter (38) umfasst, der über ein Drehelement (51) mit einem Trägerrahmen (21) verbunden ist, wobei der Schnellwechseladapter (38) eingerichtet ist, in der Aufnahme (382) über eine Drehachse des Drehelementes (51) von einer ersten Position in eine zweite Position schwenkbar zu sein, wobei der Schnellwechseladapter (38) in der zweiten Position so aus der Aufnahme (382) herausgeschwenkt ist, dass in der zweiten Position eine Aufnahme (383) für eine O2 Kartusche zugänglich ist und eine O2 Kartusche eingeführt werden kann und wobei der Schnellwechseladapter (38) dann zusammen mit der O2-Kartusche in die erste Position in die Aufnahme (382) zurück geschwenkt werden kann.

16. Vorrichtung (10) nach einem der Ansprüche 4 bis 15, **dadurch gekennzeichnet, dass** die Benutzerschnittstelle (47) der Vorrichtung (10) als Bedien-/und Anzeigeelement (49) eingerichtet und ausgebildet ist, wobei das Bedien-/und Anzeigeelement (49) in einer Fläche des Gehäuses (11) der Vorrichtung (10) ausgebildet ist, wobei das Bedien-/und Anzeigeelement (49) so großflächig ausgebildet ist, dass es mehr als 45 % oder bevorzugt mehr als 50 % dieser Fläche des Gehäuses (11), insbesondere der Dachwand (24), einnimmt.

17. Vorrichtung (10) nach Anspruch 16, **dadurch gekennzeichnet, dass** das Bedien-/und Anzeigeelement (49) in einer Fläche des Gehäuses (11) der Vorrichtung (10), insbesondere der Dachwand (24), ausgebildet ist, wobei das Bedien-/und Anzeigeelement (49) eingerichtet ist, eine Darstellung wiederzugeben, wobei die Ausrichtung der Darstellung in Abhängigkeit der horizontalen oder vertikalen Ausrichtung der Vorrichtung, entsprechend der gewählten Bodenwand (26, 27), in geänderter Orientierung erfolgt.

18. Vorrichtung (10) nach zumindest einem der Ansprüche 16 oder 17, **dadurch gekennzeichnet, dass** das Bedien-/und Anzeigeelement (49) eine annähernd vollständige Fläche des Gehäuses (11) ausbildet, wobei das Bedien-/und Anzeigeelement (49) über Dichtungen (48) mit Seitenwänden (26) des Gehäuses (19) verbunden ist, wobei zumindest eine Dichtung (48) eingerichtet und ausgebildet ist, Schnittstellenelemente und Anschlüsse aufzunehmen.

19. Vorrichtung (10) nach zumindest einem der Ansprüche 16 bis 18, **dadurch gekennzeichnet, dass** das Bedien-/ und Anzeigeelement (49) eingerichtet ist, eine Umgebungshelligkeit zu erfassen und basierend auf der erfassten Umgebungshelligkeit eine Änderung einer optischen Anzeige des Bedien-/und Anzeigeelementes (49) vorzunehmen, wobei das Bedien-/ und Anzeigeelement (49) eingerichtet ist, bei einer hellen erfassten Umgehungshelligkeit eine Farbintensität zu ändern oder von einer farbigen Anzeige auf eine schwarz-weiße Anzeige zu wechseln.

20. Vorrichtung (10) nach zumindest einem der voranstehenden Ansprüche, **dadurch gekennzeichnet, dass** die Vorrichtung (10) eine Alarmeinheit (50) mit einem Lautsprecher umfasst, die eingerichtet ist, bei erkannten Vorkommnissen einen Alarm auszugeben, wobei die Vorrichtung (10) zumindest ein Mikrophon umfasst, dass eingerichtet ist, ein von der Alarmeinheit (50) ausgegebenen Alarm zu überwachen.

21. Vorrichtung (10) nach zumindest einem der voranstehenden Ansprüche, **dadurch gekennzeichnet, dass** der Kühllüfter (17) regelbar eingerichtet ist, wobei eine Drehzahl des Kühllüfters (17) in Abhängigkeit einer erfassten Temperatur zumindest einer Komponente der Vorrichtung regelbar ist.

22. Vorrichtung (10) Anspruch 7 oder nach zumindest einem der voranstehenden Ansprüche wenn von Anspruch 7 abhängig, **dadurch gekennzeichnet, dass** die Vorrichtung (10) eingerichtet ist, einen Ladestrom des Akkumulators (16) zu regeln, wobei der Ladestrom des Akkumulators (16) in Abhängigkeit einer erfassten Temperatur des Akkumulators (16), des Kühllüfters (17) oder zumindest einer weiteren Komponente der Vorrichtung (10) regelbar ist.

## Claims

1. A respiratory device (10) comprising a housing (11), a user interface (47), and a pneumatic unit (12), wherein the pneumatic unit (12) comprises at least one of each of the components respiratory gas drive (13), measuring unit (14), control unit (32), and sound unit (22), wherein the pneumatic unit (12) is designed as a pneumatic conveying line (18), wherein the pneumatic conveying line (18) forms a respiratory gas path (B) from a device inlet (19) to a device outlet (20) of the housing (11) and wherein at least one component of the pneumatic unit (12) or the pneumatic conveying line (18) can be removed from the housing (11), wherein a cooling air path, driven by a cooling fan (17), is guided through the housing (11) from a cooling air inlet (31) to a cooling air outlet (33), wherein at least the control unit (32), the respiratory gas drive (13), and a main circuit board (40) are arranged in the cooling air path, **characterized in that** the control unit (32), the respiratory gas drive (13), and the main circuit board (40) are arranged in the cooling air path such that the component having the highest heat build-up during operation is arranged at the end of the cooling air path in the direction of flow of the cooling air path and the component having the highest heat build-up during operation is the respiratory gas drive.

2. The respiratory device (10) according to claim 2, **characterized in that** the pneumatic conveying line (18) is formed and arranged from the device inlet (19) to the device outlet (20) of the housing (11) and comprises a support frame (21) on which at least one component, in particular the respiratory gas drive (13), at least the sound unit (22), at least one flow measurement path (23), at least the measuring unit (14), and the cooling fan (17) are arranged, wherein the pneumatic conveying line (18) can be removed from the housing (11), wherein the pneumatic conveying line (18) is mounted in the housing (11) of the device (10) by at least one viscoelastic or elastomeric suspension (34).

3. The device (10) according to one of the preceding claims, **characterized in that** the respiratory gas path (B) is pneumatically separate from the cooling air path (A) and/or that the device inlet (19) is arranged in the housing (11) in a manner spaced apart from the cooling air outlet (33) such that no air from the cooling air outlet (33) is aspirated through the device inlet (19).

4. The device (10) according to claim 1, **characterized in that** the housing (11) has a top wall (24), at least one bottom wall (25), and at least two side walls (26), wherein the device inlet (19) and the device outlet (20) are formed on two non-opposite side walls (26) of the housing (11).

5. The device (10) according to one of the preceding claims, **characterized in that** at least one side wall (26) is configured as an optional bottom wall (27).

6. The device (10) according to claim 5, **characterized in that** the bottom wall (27) of the housing (11) is detachable and the pneumatic conveying line (18) is removable in one piece through an opening in the bottom wall (27).

7. The device (10) according to one of claims 5 or 6, **characterized in that** an accumulator (16) and the respiratory gas drive (13) are arranged in the housing (11) such that, in an orientation of the device (10) both on the bottom wall (25) and on the side wall (26) configured as an optional bottom wall (27), a center of gravity is formed by the accumulator (16) and the respiratory gas drive (13).

8. The device (10) according to one of claims 5 to 7, **characterized in that** no cooling air inlet (31) or cooling air outlet (33) or device inlet (19) or device outlet (20) is arranged either in the bottom wall (25) or in the side wall (26) configured as an optional bottom wall (27).

9. The device (10) according to one of the preceding claims, **characterized in that** the pneumatic conveying line (18) has at least two sound units (28a, 28b, 28c), wherein at least one sound unit (28a, 28b) is arranged on a suction side (29) of the pneumatic conveying line (18) and one sound unit (28c) is arranged on a pressure side (30) of the pneumatic conveying line (18), wherein the sound unit (22) on the suction side (29) is arranged on a receptacle of the respiratory gas drive (13) and comprises a blower cap (41), a foam carrier (42), at least one absorber foam (43), a decoupling device (44), and at least one ring foam (45).

10. The device (10) according to one of the preceding claims, **characterized in that** the device (10) has a cooling unit (39), wherein the cooling unit (39) has the cooling air inlet (31) and the cooling air outlet (33), wherein the cooling fan (17) is arranged in the housing (11) such that an air flow from the cooling air inlet (31) is guided at least via the control unit (32) and the respiratory gas drive (13) in the direction of the cooling air outlet (33).

11. The device (10) according to one of the preceding claims, **characterized in that** the cooling air path (A) is designed and configured in an opposite direction to and independent of the respiratory gas path of the pneumatic conveying line (18).

12. The device (10) according to one of the preceding claims, **characterized in that** the pneumatic unit (12) comprises a flow measurement path (23), which comprises at least one measuring unit (14) and is configured to detect at least one parameter of a respiratory gas in the flow measurement path (23), wherein the flow measurement path (23) is designed and configured linearly, wherein a lattice mesh is arranged at a start and/or at an end of the flow measurement path (23), and wherein the flow measurement path (23) is designed to be removable and reconditionable.

13. The device (10) according to claim 12, **characterized in that** the at least one measuring unit (14) is designed and configured to be removable from the housing (11), wherein the at least one measuring unit (14) comprises at least one sensor (52) for detecting at least one parameter of the respiratory air guided via the respiratory gas path.

14. The device (10) according to one of the preceding claims, **characterized in that** an expiration unit (15) is designed to be removable from a receptacle (151) of the housing (11), wherein the expiration unit (15) comprises at least one PEEP valve which has a diaphragm (154) to which a control pressure can be applied in order to block or release a respiratory gas flow through the expiration unit and/or wherein the expiration unit (15) has at least one tap (155) for a flow measurement.

15. The device (10) according to one of the preceding claims, **characterized in that** the device (10) has a receptacle (382) for a quick-change adapter and comprises a quick-change adapter (38) which is connected to a support frame (21) by a rotary element (51), wherein the quick-change adapter (38) is configured to be pivotable in the receptacle (382) from a first position to a second position via an axis of rotation of the rotary element (51), wherein the quick-change adapter (38) in the second position is pivoted out of the receptacle (382) so that, in the second position, a receptacle (383) for an O2 cartridge is accessible and an O2 cartridge can be inserted, and wherein the quick-change adapter (38) can then be pivoted back into the receptacle (382), together with the O2 cartridge, to the first position.

16. The device (10) according to one of claims 4 to 15, **characterized in that** the user interface (47) of the device (10) is configured and designed as an operating and display element (49), wherein the operating and display element (49) is designed in a face of the housing (11) of the device (10), wherein the operating and display element (49) is designed with a large area so that it takes up more than 45% or preferably more than 50% of this face of the housing (11), in particular the top wall (24).

17. The device (10) according to claim 16, **characterized in that** the operating and display element (49) is designed in a face of the housing (11) of the device (10), in particular the top wall (24), wherein the operating and display element (49) is configured to display a representation, wherein the orientation of the representation takes place depending on the horizontal or vertical orientation of the device, corresponding to the selected bottom wall (26, 27), in a modified orientation.

18. The device (10) according to at least one of claims 16 or 17, **characterized in that** the operating and display element (49) forms a nearly complete face of the housing (11), wherein the operating and display element (49) is connected to side walls (26) of the housing (19) via seals (48), wherein at least one seal (48) is configured and designed to receive interface elements and connections.

19. The device (10) according to at least one of claims 16 to 18, **characterized in that** the operating and display element (49) is configured to detect an ambient brightness and make a change of an optical display of the operating and display element (49) based on the detected ambient brightness, wherein the operating and display element (49) is configured to change a color intensity or to change from a color display to a black and white display in the case of a bright detected ambient brightness.

20. The device (10) according to at least one of the preceding claims, **characterized in that** that device (10) comprises an alarm unit (50) with a speaker, which is configured to emit an alarm in the case of recognized incidents, wherein the device (10) comprises at least one microphone which is configured to monitor an alarm emitted by the alarm unit (50).

21. The device (10) according to at least one of the preceding claims, **characterized in that** the cooling fan (17) is configured to be regulatable, wherein a rotational speed of the cooling fan (17) can be regulated depending on a detected temperature of at least one component of the device.

22. The device (10) according to claim 7 or according to at least one of the preceding claims if dependent on claim 7, **characterized in that** the device (10) is configured to regulate a charging current of the accumulator (16), wherein the charging current of the accumulator (16) can be regulated depending on a detected temperature of the accumulator (16), the cooling fan (17), or at least one further component of the device (10).

## Revendications

1. Dispositif (10) de respiration comportant un boîtier (11), une interface d'utilisateur (47) et une unité pneumatique (12), dans lequel l'unité pneumatique (12) comporte au moins l'un des composants parmi un actionneur de gaz respiratoire (13), une unité de mesure (14), une unité de commande (32) et une unité acoustique (22), dans lequel l'unité pneumatique (12) est conçue comme un canal pneumatique (18), dans lequel le canal pneumatique (18) forme un circuit de gaz respiratoire (B) s'étendant d'une entrée d'appareil (19) jusqu'à une sortie d'appareil (20) du boîtier (11) et dans lequel au moins un composant de l'unité pneumatique (12) ou du canal pneumatique (18) peut être retiré du boîtier (11), dans lequel un circuit d'air de refroidissement, commandé par un ventilateur de refroidissement (17), s'étend à partir d'une entrée d'air de refroidissement (31) jusqu'à une sortie d'air de refroidissement (33) à travers le boîtier (11), dans lequel au moins l'unité de commande (32), l'actionneur de gaz respiratoire (13) et une platine principale (40) sont disposés dans le circuit d'air de refroidissement, **caractérisé en ce que** l'unité de commande (32), l'actionneur de gaz respiratoire (13) et la platine principale (40) sont disposés de telle façon dans le circuit d'air de refroidissement, que le composant produisant le plus de chaleur pendant le fonctionnement est disposé à la fin du circuit d'air de refroidissement dans la direction d'écoulement du circuit d'air de refroidissement et le composant produisant le plus de chaleur pendant le fonctionnement est l'actionneur de gaz respiratoire.

2. Dispositif (10) de respiration selon la revendication 2, **caractérisé en ce que** le canal pneumatique (18) est formé et disposé entre l'entrée d'appareil (19) et la sortie d'appareil (20) du boîtier (11) et comporte un cadre de support (21), sur lequel est disposé au moins un composant, en particulier l'actionneur de gaz respiratoire (13), au moins l'unité acoustique (22), au moins un trajet de mesure du débit (23), au moins l'unité de mesure (14) et le ventilateur de refroidissement (17), dans lequel le canal pneumatique (18) peut être retiré du boîtier (11), dans lequel le canal pneumatique (18) est maintenu par au moins une suspension visco-élastique ou élastomère (34) dans le boîtier (11) du dispositif (10).

3. Dispositif (10) selon l'une des revendications précédentes, **caractérisé en ce que** le circuit de gaz respiratoire (B) est séparé pneumatiquement du circuit d'air de refroidissement (A) et/ou **en ce que** l'entrée d'appareil (19) est espacée de telle façon de la sortie d'air de refroidissement (33) dans le boîtier (11), qu'aucun air n'est aspiré par l'entrée d'appareil (19) à partir de la sortie d'air de refroidissement (33).

4. Dispositif (10) selon la revendication 1, **caractérisé en ce que** le boîtier (11) présente une paroi supérieure (24), au moins une paroi inférieure (25) et au moins deux parois latérales (26), dans lequel l'entrée d'appareil (19) et la sortie d'appareil (20) sont conçues sur deux parois latérales (26) du boîtier (11) non opposées l'une à l'autre.

5. Dispositif (10) selon l'une des revendications précédentes, **caractérisé en ce qu'**au moins une paroi latérale (26) est configurée comme une paroi inférieure (27) facultative.

6. Dispositif (10) selon la revendication 5, **caractérisé en ce que** la paroi inférieure (27) du boîtier (11) est amovible et le canal pneumatique (18) peut être retiré en une seule pièce par une ouverture de la paroi inférieure (27).

7. Dispositif (10) selon la revendication 5 ou 6, **caractérisé en ce qu'**un accumulateur (16) et l'actionneur de gaz respiratoire (13) sont disposés de telle façon dans le boîtier (11), que lors d'une orientation du dispositif (10) aussi bien que la paroi inférieure (25) que sur la paroi latérale (26) configurée comme une paroi inférieure facultative (27), un centre de gravité est formé par l'accumulateur (16) et l'actionneur de gaz respiratoire (13).

8. Dispositif (10) selon l'une des revendications 5 à 7, **caractérisé en ce qu'**aucune entrée d'air de refroidissement (31) ni sortie d'air de refroidissement (33) ou entrée d'appareil (19) ni sortie d'appareil (20) n'est disposée dans la paroi inférieure (25) ou dans la paroi latérale (26) configurée comme une paroi inférieure facultative (27).

9. Dispositif (10) selon l'une des revendications précédentes, **caractérisé en ce que** le canal pneumatique (18) présente au moins deux unités acoustiques (28a, 28b, 28c), dans lequel au moins une unité acoustique (28a, 28b) est disposée sur un côté aspiration (29) du canal pneumatique (18) et une unité acoustique (28c) est disposée sur un côté pression (30) du canal pneumatique (18), dans lequel l'unité acoustique (22) sur le côté aspiration (29) est disposée sur un logement de l'actionneur de gaz respiratoire (13) et comporte un capot de soufflerie (41), un support de mousse (42), au moins une mousse absorbante (43), un dispositif de désaccouplement (44) et au moins une mousse annulaire (45).

10. Dispositif (10) selon l'une des revendications précédentes, **caractérisé en ce que** le dispositif (10) présente une unité de refroidissement (39), dans lequel l'unité de refroidissement (39) présente l'entrée d'air de refroidissement (31) et la sortie d'air de refroidissement (33), dans lequel le ventilateur de refroidissement (17) est disposé de telle façon dans le boîtier (11), qu'un flux d'air est guidé à partir de l'entrée d'air de refroidissement (31) vers la sortie d'air de refroidissement (33) en passant au moins par l'unité de commande (32) et l'actionneur de gaz respiratoire (13).

11. Dispositif (10) selon l'une des revendications précédentes, **caractérisé en ce que** le circuit d'air de refroidissement (A) est conçu et configuré à contre-courant du circuit de gaz respiratoire du canal pneumatique (18) et indépendamment de celui-ci.

12. Dispositif (10) selon l'une des revendications précédentes, **caractérisé en ce que** l'unité pneumatique (12) comporte un trajet de mesure du débit (23) comportant au moins une unité de mesure (14) et configuré pour détecter au moins un paramètre d'un gaz respiratoire sur le trajet de mesure du débit (23), dans lequel le trajet de mesure du débit (23) est réalisé et configuré de façon linéaire, dans lequel une grille est disposée au niveau d'un début et/ou d'une fin du trajet de mesure du débit (23) et dans lequel le trajet de mesure du débit (23) est conçu de manière à pouvoir être retiré et préparé.

13. Dispositif (10) selon la revendication 12, **caractérisé en ce que** l'au moins une unité de mesure (14) est conçue et configurée de manière à pouvoir être retirée du boîtier (11), dans lequel l'au moins une unité de mesure (14) comporte au moins un capteur (52) destiné à détecter au moins un paramètre de l'air respiratoire acheminé par le biais du circuit de gaz respiratoire.

14. Dispositif (10) selon l'une des revendications précédentes, **caractérisé en ce qu'**une unité d'expiration (15) est conçue de manière à pouvoir être retirée d'un logement (151) du boîtier (11), dans lequel l'unité d'expiration (15) comporte au moins une soupape PEEP présentant une membrane (154), laquelle peut être soumise à une pression de commande, pour bloquer ou libérer un flux de gaz respiratoire par le biais de l'unité d'expiration et/ou dans lequel l'unité d'expiration (15) présente au moins une prise (155) pour une mesure de flux.

15. Dispositif (10) selon l'une des revendications précédentes, **caractérisé en ce que** le dispositif (10) présente un logement (382) pour un adaptateur de remplacement rapide et comporte un adaptateur de remplacement rapide (38), lequel est relié à un cadre de support (21) par le biais d'un élément rotatif (51), dans lequel l'adaptateur de remplacement rapide (38) est configuré de manière à pouvoir pivoter dans le logement (382) d'une première position vers une deuxième position autour d'un axe de rotation de l'élément rotatif (51), dans lequel, dans la deuxième position, l'adaptateur de remplacement rapide (38) est pivoté hors du logement (382) de manière à permettre l'accès à un logement (383) pour une cartouche d'O2 et l'introduction d'une cartouche d'O2 dans la deuxième position et dans lequel l'adaptateur de remplacement rapide (38) peut ensuite pivoter de nouveau vers la première position dans le logement (382) conjointement avec la cartouche d'O2.

16. Dispositif (10) selon l'une des revendications 4 à 15, **caractérisé en ce que** l'interface d'utilisateur (47) du dispositif (10) est configurée et conçue comme un élément de commande et d'affichage (49), dans lequel l'élément de commande et d'affichage (49) est conçu sur une surface du boîtier (11) du dispositif (10), dans lequel l'élément de commande et d'affichage (49) est conçu sur une surface d'une taille telle qu'il occupe plus de 45% ou de préférence plus de 50% de cette surface du boîtier (11), en particulier de la paroi supérieure (24).

17. Dispositif (10) selon la revendication 16, **caractérisé en ce que** l'élément de commande et d'affichage (49) est conçu sur une surface du boîtier (11) du dispositif (10), en particulier sur la paroi supérieure (24), dans lequel l'élément de commande et d'affichage (49) est configuré pour reproduire une représentation, dans lequel l'orientation de la représentation se fait dans un sens modifié en fonction de l'orientation horizontale ou verticale du dispositif, selon la paroi inférieure (26, 27) choisie.

18. Dispositif (10) selon l'une au moins des revendications 16 ou 17, **caractérisé en ce que** l'élément de commande et d'affichage (49) forme une surface quasiment complète du boîtier (11), dans lequel l'élément de commande et d'affichage (49) est relié à des parois latérales (26) du boîtier (19) par des joints (48), dans lequel au moins un joint (48) est configuré et conçu pour recevoir des éléments d'interface et des raccords.

19. Dispositif (10) selon l'une au moins des revendications 16 à 18, **caractérisé en ce que** l'élément de commande et d'affichage (49) est configuré pour détecter une luminosité ambiante et pour modifier un affichage optique de l'élément de commande et d'affichage (49) sur la base de la luminosité ambiante détectée, dans lequel l'élément de commande et d'affichage (49) est configuré pour modifier une intensité de couleur ou pour passer d'un affichage en couleur à un affichage en noir et blanc en présence d'une luminosité ambiante claire détectée.

20. Dispositif (10) selon l'une au moins des revendications précédentes, **caractérisé en ce que** le dispositif (10) comporte une unité d'alarme (50) avec un haut-parleur, laquelle est configurée pour émettre une alarme lors de la détection d'événements, dans lequel le dispositif (10) comporte au moins un microphone, lequel est configuré pour surveiller une alarme émise par l'unité d'alarme (50).

21. Dispositif (10) selon l'une au moins des revendications précédentes, **caractérisé en ce que** le ventilateur de refroidissement (17) est configuré de manière à pouvoir être réglé, dans lequel une vitesse de rotation du ventilateur de refroidissement (17) peut être réglée en fonction d'une température détectée d'au moins un composant du dispositif.

22. Dispositif (10) selon la revendication 7 ou selon l'une au moins des revendications précédentes dans la mesure où celle-ci dépend de la revendication 7, **caractérisé en ce que** le dispositif (10) est configuré pour régler un courant de charge de l'accumulateur (16), dans lequel le courant de charge de l'accumulateur (16) peut être réglé en fonction d'une température détectée de l'accumulateur (16), du ventilateur de refroidissement (17) ou d'au moins un autre composant du dispositif (10).
